# EUROPEAN PATENT SPECIFICATION

(11) **EP 3 586 848 B1**
(45) Date of publication and mention of the grant of the patent: **01.09.2021**
(21) Application number: 18758340.6
(22) Date of filing: 23.02.2018
(51) Int. Cl.: A61K 31/505, A61K 31/506, A61K 31/437, A61K 31/415, A61K 31/519, A61K 31/5377, A23L 33/10, A61K 31/5513, A61K 39/00

(54) **PHARMACEUTICAL COMPOSITION COMPRISING COMPOUND CAPABLE OF PENETRATING BLOOD-BRAIN BARRIER AS EFFECTIVE INGREDIENT FOR PREVENTING OR TREATING BRAIN CANCER**
PHARMAZEUTISCHE ZUSAMMENSETZUNG MIT ZUR PENETRIERUNG DER BLUT-HIRN-SCHRANKE FÄHIGER VERBINDUNG ALS WIRKSTOFF ZUR PRÄVENTION ODER BEHANDLUNG VON GEHIRNKREBS
COMPOSITION PHARMACEUTIQUE COMPRENANT UN COMPOSÉ APTE À TRAVERSER LA BARRIÈRE HÉMATO-ENCÉPHALIQUE EN TANT QUE PRINCIPE ACTIF POUR PRÉVENIR OU TRAITER LE CANCER DU CERVEAU

(30) Priority: 24.02.2017 KR 20170024812
(43) Date of publication of application: 01.01.2020
(73) Proprietor: Daegu-Gyeongbuk Medical Innovation Foundation, Daegu 41061 (KR); National Cancer Center, Goyang-si, Gyeonggi-do 10408 (KR); Samsung Life Public Welfare Foundation, Seoul 04348 (KR)
(72) Inventor: CHOI, Hwan Geun, Seoul 06548 (KR); PARK, Jong Bae, Goyang-si Gyeonggi-do 10325 (KR); KO, Eunhwa, Daegu 41090 (KR); SON, Jung Beom, Daegu 41061 (KR); KIM, Nam Doo, Daegu 42214 (KR); LEE, Seung Hoon, Daejeon 35233 (KR); NAM, Do-Hyun, Seoul 06351 (KR)
(74) Representative: Office Freylinger
(86) International application number: PCT/KR2018/002241
(87) International publication number: WO 2018/155947

(56) References cited:
- WO-A1-2011/038572
- WO-A1-2011/141756
- WO-A1-2012/162254
- WO-A1-2014/106612
- WO-A1-2014/160430
- WO-A1-2014/170248
- US-A1- 2013 338 106
- US-A1- 2014 206 683
- HWAN GEUN CHOI ET AL: "Brain Penetrant LRRK2 Inhibitor", ACS MEDICINAL CHEMISTRY LETTERS, AMERICAN CHEMICAL SOCIETY, US, vol. 3, no. 8, 9 August 2012 (2012-08-09), pages 658-662, XP009166203, ISSN: 1948-5875, DOI: 10.1021/ML300123A [retrieved on 2012-06-18]
- ANTHONY A. ESTRADA ET AL: "Discovery of Highly Potent, Selective, and Brain-Penetrable Leucine-Rich Repeat Kinase 2 (LRRK2) Small Molecule Inhibitors", JOURNAL OF MEDICINAL CHEMISTRY, vol. 55, no. 22, 26 November 2012 (2012-11-26), pages 9416-9433, XP055048309, ISSN: 0022-2623, DOI: 10.1021/jm301020q

## Description

### Technical Field

The present invention relates to a pharmaceutical composition for the prevention or treatment of brain cancer, containing, as an active ingredient, a compound capable of penetrating a blood-brain barrier.

### Background Art

Protein kinase is an enzyme that catalyzes the transfer of the terminal phosphate group of adenosine triphosphate (ATP) to tyrosine, serine or threonine, which is a specific residue of protein, and this enzyme is involved in signals that regulate cell activity or growth and differentiation depending on extracellular media and environmental changes.

Inappropriately high protein kinase activity is directly or indirectly related to a number of diseases resulting from abnormal cellular actions. For example, disease may be caused by failure of the appropriate regulatory mechanism of kinase involved in mutation, overexpression or unsuitable enzyme activity, or by excessive production or lack of factors involved in upstream or downstream signaling of cytokines and kinases. Thus, selective inhibition of kinase activity may become a goal for furthering the development of new drugs for the treatment of disease.

The LRRK2 (leucin-rich repeat kinase-2) protein belongs to the leucine-rich repeat kinase family and is composed of a 2527-amino-acid sequence having high interspecies similarity. The LRRK2 protein is characterized by exhibiting both GTPase and serine-threonine kinase activities in a single protein. The expressed LRRK2 protein is observed in a variety of organs and tissues, including the brain, and is present in the cytoplasm or cell membrane and mitochondrial outer membrane at the cellular level.

Furthermore, the LRRK2 protein has five functionally important domains and is inferred to regulate cell function through protein interaction and enzymatic action, as well as autoregulatory action through autophosphorylation. In particular, chaperone machinery, cytoskeleton arrangement, protein translational machinery, synaptic vesicle endocytosis, mitogen-activated protein-kinase-signaling cascades and ubiquitin/autophage protein degradation pathways are known to be regulated by the LRRK2 protein.

The LRRK2 protein has been reported to be associated with Alzheimer's-disease-related mild cognitive impairment progression, L-dopa-induced dyskinesia, and neural-precursor-differentiation-related CNS disorder. Furthermore, the G2019S mutation of the LRRK2 protein has been reported to increase the incidence of non-skin cancer such as acute myeloid leukemia (AML), kidney cancer, breast cancer, lung cancer, prostate cancer and the like. Specifically, the G2019S mutation of the LRRK2 protein increases the catalytic activity of the LRRK2 kinase domain. Moreover, it has been reported that the LRRK2 protein is also associated with amyotrophic lateral sclerosis, rheumatoid arthritis, and ankylosing spondylitis (International Patent Publication No. WO 2011/038572).

WO 2014/160430 discloses small molecule LRRK2 and ERK5 inhibitors, as well as methods for their use in treating or preventing cancer and/or neurodegenerative disorders. WO 2014/170248 relates to arylpyrrolopyridine derivatives which are LRRK2 inhibitors and thus useful in therapy and to pharmaceutical composition comprising said compounds. WO 2014/106612 relates to aminopyridine derivatives which are LRRK2 inhibitors and thus useful in therapy and to pharmaceutical composition comprising said compounds.

Meanwhile, methods of treating brain tumors include surgery, radiotherapy, anticancer drug therapy (chemotherapy), and hormone therapy. Among these, surgery and radiotherapy have been mainly carried out, but radiotherapy has a problem in that it is accompanied by side effects such as neurotoxicity and dementia. With the goal of solving such problems, anticancer drug therapy (chemotherapy) is receiving attention, but drug therapy for the treatment of brain tumors is problematic in that most drugs are blocked by a special protection system called the 'blood-brain barrier' (BBB) and thus cannot be delivered to the brain, and is limited due to the resistance of cancer to anticancer drugs.

Therefore, the present inventors first proved through *in-vivo* experiments that a compound, serving as an LRRK2 protein inhibitor, may efficiently penetrate the blood-brain barrier (BBB) and is thus useful for the prevention and treatment of brain cancer, which culminates in the present invention.

### Disclosure

### Technical Problem

An objective of the present invention is to provide a pharmaceutical composition, which is capable of efficiently penetrating the blood-brain barrier and is thus useful in the prevention or treatment of brain cancer.

### Technical Solution

In order to accomplish the above objective, the present invention provides a pharmaceutical composition for use in the prevention or treatment of brain cancer, containing an LRRK2 protein inhibitor selected from the following compound group as an active ingredient:
(1) 5-chloro-N4-(2-(isopropylsulfonyl)phenyl)-N2-(2-methoxy-4-(4-(4-methylpiperazin-1-yl)piperidin-1-yl)phenyl)pyrimidine-2,4-diamine; (10) 2-[(2-methoxy-4-[[4-(4-methylpiperazin-1-yl)piperidin-1-yl]carbonyl]phenyl)amino]-5,11-dimethyl-5,11-dihydro-6H-pyrimido[4,5-b][1,4]benzodiazepin-6-one; (11) (4-(4-(ethylamino)-5-(trifluoromethyl)pyrimidin-2-ylamino)-2-fluoro-5-methoxyphenyl)(morpholino)methanone; (12) (3-methoxy-4-(4-(methylamino)-5-(trifluoromethyl)pyrimidin-2-ylamino)phenyl)(morpholino)methanone; (20) (2S,6R)-2,6-dimethyl-4-(6-(5-(1-methylcyclopropoxy)-1H-indazol-3-yl)pyrimidin-4-yl)morpholine; (21) 3-(4-morpholino-1H-pyrrolo[2,3-b]pyridin-3-yl)benzonitrile; and (22) 1-methyl-4-(4-morpholino-7H-pyrrolo[2,3-d]pyrimidin-5-yl)-1H-pyrrole-2-carbonitrile.

In addition, the present invention provides a pharmaceutical kit for use in the prevention or treatment of brain cancer, including a first component containing a pharmaceutically effective amount of an LRRK2 protein inhibitor selected from the following compound group as an active ingredient:
(1) 5-chloro-N4-(2-(isopropylsulfonyl)phenyl)-N2-(2-methoxy-4-(4-(4-methylpiperazin-1-yl)piperidin-1-yl)phenyl)pyrimidine-2,4-diamine; (10) 2-[(2-methoxy-4-[[4-(4-methylpiperazin-1-yl)piperidin-1-yl]carbonyl]phenyl)amino]-5,11-dimethyl-5,11-dihydro-6H-pyrimido[4,5-b][1,4]benzodiazepin-6-one; (11) (4-(4-(ethylamino)-5-(trifluoromethyl)pyrimidin-2-ylamino)-2-fluoro-5-methoxyphenyl)(morpholino)methanone; (12) (3-methoxy-4-(4-(methylamino)-5-(trifluoromethyl)pyrimidin-2-ylamino)phenyl)(morpholino)methanone; (20) (2S,6R)-2,6-dimethyl-4-(6-(5-(1-methylcyclopropoxy)-1H-indazol-3-yl)pyrimidin-4-yl)morpholine; (21) 3-(4-morpholino-1H-pyrrolo[2,3-b]pyridin-3-yl)benzonitrile; and (22) 1-methyl-4-(4-morpholino-7H-pyrrolo[2,3-d]pyrimidin-5-yl)-1H-pyrrole-2-carbonitrile;
and a second component containing a pharmaceutically effective amount of Avastin® (bevacizumab) as an active ingredient.

### Advantageous Effects

According to the present invention, a pharmaceutical composition for use in the prevention or treatment of brain cancer contains an LRRK2 protein inhibitor as an active ingredient, and is thus effective in significantly inhibiting tumor formation by easily penetrating the blood-brain barrier, through which general drugs are difficult to pass.

### Description of Drawings

FIG. 1 is images showing the results of Western blotting of LRRK2 protein expression in cancer tissue obtained from brain tumor patients.
FIG. 2 is images showing the results of evaluation of the correlation between LRRK2 protein phosphorylation and brain tumor malignancy.
FIG. 3 is an image showing the results of analysis of a nucleic acid sequence encoding the LRRK2 protein expressed in brain tumor cells.
FIG. 4a schematically shows the structure of a lentiviral vector expressing the LRRK2 protein.
FIG. 4b is images showing the overexpression of the LRRK2 protein in cells infected with a lentiviral vector expressing the LRRK2 protein.
FIG. 4c is images showing the self-renewal of tumor cells in cells infected with a lentiviral vector expressing the LRRK2 protein.
FIG. 5a is images showing the results of Western blotting upon treatment of brain-tumor-patient-derived cell line NCC01 cells with the compounds of Examples 10, 11, 12, 20 and 21.
FIG. 5b is images showing the results of Western blotting upon treatment of brain-tumor-patient-derived cell line NCC01 cells with the compounds of Examples 11, 12, 20, 21 and 22.
FIG. 6 is a graph showing the results of Western blotting upon treatment of brain-tumor-patient-derived cell line NCC01 cells with the compounds of Examples 10, 11, 12, 20 and 21.
FIGS. 7 and 8 are images showing the results of Western blotting upon treatment of brain-tumor-patient-derived cell line NCC01 cells with the compound of Example 10 at various concentrations.
FIGS. 9 and 10 are images showing the results of Western blotting upon treatment of brain-tumor-patient-derived cell line NCC01 cells with the compound of Example 12 at various concentrations.
FIGS. 11 and 12 are images showing the results of Western blotting over time after treatment of brain-tumor-patient-derived cell line NCC01 cells with the compound of Example 12 at a concentration of 100 nM.
FIGS. 13 to 15 are images showing the results of Western blotting 1 day after treatment of NCC01 cells with the compound of Example 12 in the concentration range of 50 nM to 2,000 nM.
FIG. 16 is images showing the death of brain tumor cells induced by inhibiting the activity of mitochondria using the compound of Example 12.
FIG. 17 is images showing the inhibition of the activity of mitochondria present in brain tumor stem cells using the compound of Example 12.
FIG. 18 is images showing the inhibition of mitochondrial activity by suppressing TRAP1 expression using the compound of Example 12.
FIG. 19a is images showing that the growth of spheres formed from NCC01 cells exposed to hypoxic conditions is inhibited by shRNA against the LRRK2 gene.
FIG. 19b is images showing the results of Western blotting of LRRK2 protein expression in NCC01 cells exposed to hypoxic conditions.
FIG. 19c is images showing that the growth of spheres formed from 528NS cells exposed to hypoxic conditions is inhibited by shRNA against the LRRK2 gene.
FIG. 19d is images showing the results of Western blotting of LRRK2 protein expression in 528NS cells exposed to hypoxic conditions.
FIGS. 20 and 21 are images showing the results of evaluation of cell cycle FACS 1 day after treatment of NCC01 cells with the compounds of Example 11, 12 and 21 at concentrations of 100 nM.
FIG. 22 is an image showing the results of observation of changes in number of days of survival after oral administration of the compound of Example 12 to a brain-tumor-induced mouse model.
FIG. 23 is images showing the result of measurement of changes in tumor size after oral administration of the compound of Example 12 to a brain-tumor-induced mouse model.
FIG. 24 is an image showing an increase in the therapeutic effect on brain tumors when the compound of Example 12 is administered in combination with Avastin.
FIG. 25 is images showing the results of Western blotting upon treatment of brain-tumor-patient-derived cell line NCC01 cells with the compound of Comparative Example.
FIG. 26 is images showing the results of Western blotting upon treatment of brain-tumor-patient-derived cell line 448T cells with the compounds of Examples 10, 11, 12, 20 and 21.
FIG. 27 is images showing the results of Western blotting upon treatment of brain-tumor-patient-derived cell line 448T cells with the compound of Example 12 at various concentrations.
FIG. 28 is an image showing the results of observation of changes in number of days of survival after oral administration of the compounds of Examples 12, 20 and 21 to a brain-tumor-induced mouse model.
FIG. 29 is images showing the results of Western blotting upon treatment of brain-tumor-patient-derived cell line 448T cells with the compound of Comparative Example and the compounds of Examples 1, 11 and 12.

Hereinafter, a detailed description will be given of the present invention.

The present invention pertains to a pharmaceutical composition for use in the prevention or treatment of brain cancer, containing an LRRK2 (leucin-rich repeat kinase-2) protein inhibitor as an active ingredient, wherein the LRRK2 protein inhibitor is any one selected from the following compound group:
(1) 5-chloro-N4-(2-(isopropylsulfonyl)phenyl)-N2-(2-methoxy-4-(4-(4-methylpiperazin-1-yl)piperidin-1-yl)phenyl)pyrimidine-2,4-diamine; (10) 2-[(2-methoxy-4-[[4-(4-methylpiperazin-1-yl)piperidin-1-yl]carbonyl]phenyl)amino]-5,11-dimethyl-5,11-dihydro-6H-pyrimido[4,5-b][1,4]benzodiazepin-6-one; (11) (4-(4-(ethylamino)-5-(trifluoromethyl)pyrimidin-2-ylamino)-2-fluoro-5-methoxyphenyl)(morpholino)methanone; (12) (3-methoxy-4-(4-(methylamino)-5-(trifluoromethyl)pyrimidin-2-ylamino)phenyl)(morpholino)methanone; (20) (2S,6R)-2,6-dimethyl-4-(6-(5-(1-methylcyclopropoxy)-1H-indazol-3-yl)pyrimidin-4-yl)morpholine; (21) 3-(4-morpholino-1H-pyrrolo[2,3-b]pyridin-3-yl)benzonitrile; and (22) 1-methyl-4-(4-morpholino-7H-pyrrolo[2,3-d]pyrimidin-5-yl)-1H-pyrrole-2-carbonitrile.

According to the present invention, the pharmaceutical composition is for use to treat brain cancer, particularly brain cancer in which the LRRK2 protein is expressed or activated. The LRRK2 protein activity may be exhibited through phosphorylation of the LRRK2 protein.

The LRRK2 protein inhibitor may be used in the form of a pharmaceutically acceptable salt thereof. Here, a useful salt may be an acid addition salt formed using a pharmaceutically acceptable free acid.

The acid addition salt may be obtained from inorganic acids such as hydrochloric acid, nitric acid, phosphoric acid, sulfuric acid, hydrobromic acid, hydroiodic acid, nitrous acid, phosphorous acid and the like, non-toxic organic acids such as aliphatic mono- and dicarboxylate, phenyl-substituted alkanoate, hydroxyalkanoate and alkanedioate, aromatic acids, aliphatic and aromatic sulfonic acids, etc., or organic acids such as acetic acid, benzoic acid, citric acid, lactic acid, maleic acid, gluconic acid, methanesulfonic acid, 4-toluenesulfonic acid, tartaric acid, fumaric acid, etc. Examples of the pharmaceutically non-toxic salt include sulfate, pyrosulfate, bisulfate, sulfite, bisulfite, nitrate, phosphate, monohydrogen phosphate, dihydrogen phosphate, metaphosphate, pyrophosphate chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexane-1,6-dioate, benzoate, chlorobenzoate, methyl benzoate, dinitrobenzoate, hydroxybenzoate, methoxybenzoate, phthalate, terephthalate, benzene sulfonate, toluene sulfonate, chlorobenzene sulfonate, xylene sulfonate, phenyl acetate, phenyl propionate, phenyl butyrate, citrate, lactate, β-hydroxybutyrate, glycolate, maleate, tartrate, methane sulfonate, propane sulfonate, naphthalene-1-sulfonate, naphthalene-2-sulfonate, mandelate, and the like.

The acid addition salt may be prepared through a typical method, for example, by dissolving an LRRK2 protein inhibitor derivative in an organic solvent such as methanol, ethanol, acetone, methylene chloride or acetonitrile and adding an organic acid or an inorganic acid thereto to give a precipitate, which is then filtered and dried, or by subjecting a solvent and an excess of acid to vacuum distillation, drying and then crystallization in the presence of an organic solvent.

Also, the LRRK2 protein inhibitor may be prepared in the form of a pharmaceutically acceptable metal salt using a base. Specifically, an alkali metal or alkaline earth metal salt may be obtained by, for example, dissolving the compound in an excess of an alkali metal hydroxide or alkaline earth metal hydroxide solution, filtering the undissolved compound salt, and evaporating the filtrate to dryness. Here, as the metal salt, a sodium salt, a potassium salt or a calcium salt is pharmaceutically appropriate. Furthermore, the corresponding salt may be obtained by reacting an alkali metal or alkaline earth metal salt with a suitable silver salt (e.g. silver nitrate).

In the pharmaceutical composition for use according to the present invention, the LRRK2 protein inhibitor, the optical isomer thereof, or the pharmaceutically acceptable salt thereof may be administered orally or parenterally in various dosage forms upon clinical administration. The formulation thereof may be prepared using diluents or excipients such as fillers, extenders, binders, wetting agents, disintegrants, surfactants and the like, which are usually used.

Examples of the formulations for oral administration include tablets, pills, hard/soft capsules, liquids, suspensions, emulsions, syrups, granules, elixirs, troches and the like. These formulations may contain diluents (such as lactose, dextrose, sucrose, mannitol, sorbitol, cellulose and/or glycine), or lubricants (such as silica, talc, stearic acid and magnesium or calcium salts thereof and/or polyethylene glycol), in addition to the active ingredient. The tablets may contain binders such as magnesium aluminum silicate, starch paste, gelatin, methyl cellulose, sodium carboxymethyl cellulose and polyvinyl pyrrolidine, and the like, and may optionally contain disintegrants such as starch, agar, alginic acid or sodium salts thereof, or boiling mixtures, absorbents, colorants, flavoring agents, and sweetening agents.

In addition, the present invention pertains to a pharmaceutical kit for use in the prevention or treatment of brain cancer, including a first component containing a pharmaceutically effective amount of an LRRK2 protein inhibitor selected from the following compound group as an active ingredient:
(1) 5-chloro-N4-(2-(isopropylsulfonyl)phenyl)-N2-(2-methoxy-4-(4-(4-methylpiperazin-1-yl)piperidin-1-yl)phenyl)pyrimidine-2,4-diamine; (10) 2-[(2-methoxy-4-[[4-(4-methylpiperazin-1-yl)piperidin-1-yl]carbonyl]phenyl) amino]-5,11-dimethyl-5, 11-dihydro-6H-pyrimido[4,5-b][1,4]benzodiazepin-6-one; (11) (4-(4-(ethylamino)-5-(trifluoromethyl)pyrimidin-2-ylamino)-2-fluoro-5-methoxyphenyl)(morpholino)methanone; (12) (3-methoxy-4-(4-(methylamino)-5-(trifluoromethyl)pyrimidin-2-ylamino)phenyl)(morpholino)methanone; (20) (2S,6R)-2,6-dimethyl-4-(6-(5-(1-methylcyclopropoxy)-1H-indazol-3-yl)pyrimidin-4-yl)morpholine; (21) 3-(4-morpholino-1H-pyrrolo[2,3-b]pyridin-3-yl)benzonitrile; and (22) 1-methyl-4-(4-morpholino-7H-pyrrolo[2,3-d]pyrimidin-5-yl)-1H-pyrrole-2-carbonitrile;
and a second component containing a pharmaceutically effective amount of Avastin® (bevacizumab) as an active ingredient.

In the pharmaceutical kit for use in the prevention or treatment of brain cancer according to the present invention, the first component and the second component may be sequentially or simultaneously administered. When the first component and the second component are sequentially administered, the second component may be administered 30 min to 180 min, 60 min to 120 min, or 80 min to 100 min before or after administration of the first component. In an embodiment of the present invention, the second component may be administered 90 min after administration of the first component.

When the first component and the second component included in the pharmaceutical kit are co-administered, the anticancer activity against brain cancer may be increased. The above administration may include administration to a mammal, particularly a human.

The pharmaceutical composition for use according to the present invention, containing, as an active ingredient, the LRRK2 protein inhibitor, the optical isomer thereof, or the pharmaceutically acceptable thereof, may be administered parenterally, and examples of parenteral administration include subcutaneous injection, intravenous injection, intramuscular injection, and intrathoracic injection.

Here, in order to produce the formulation for parenteral administration, the LRRK2 protein inhibitor, the optical isomer thereof, or the pharmaceutically acceptable salt thereof is mixed with water, along with a stabilizer or a buffer, to afford a solution or suspension, which is prepared in an ampoule or vial unit dosage form. The composition may be sterilized, and may further contain an adjuvant, such as a preservative, a stabilizer, a hydration or emulsification accelerator, a salt for controlling osmotic pressure, and a buffer, as well as other therapeutically useful substances, and may be formulated through typical mixing, granulating or coating.

Moreover, the dose of the pharmaceutical composition for use according to the present invention, containing the LRRK2 protein inhibitor, the optical isomer thereof, or the pharmaceutically acceptable salt thereof as the active ingredient, administered to a human may vary depending on the patient's age, body weight, gender, dosage form, state of health and severity of disease, and is typically 0.1 to 1000 mg/day, and preferably 1 to 500 mg/day based on an adult patient weighing 70 kg, and moreover, may be administered once or several times a day at regular time intervals, as prescribed by a doctor or pharmacist.

The present inventors have first proved that the LRRK2 protein inhibitor is effective in significantly inhibiting tumor formation by easily penetrating the blood-brain barrier through which general drugs are difficult to pass. The experimental results therefor will be described in detail through the following experimental examples.

### Mode for Invention

A better understanding of the present invention will be given through the following examples and experimental examples.

### <Example 1> Preparation of 5-chloro-N4-(2-(isopropylsulfonyl)phenyl)-N2-(2-methoxy-4-(4-(4-methylpiperazin-1-yl)piperidin-1-yl)phenyl)pyrimidine-2,4-diamine

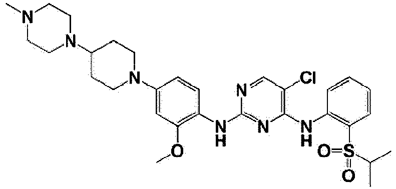

The above compound was prepared with reference to WO 2004080980, WO 2005016894, WO 2009102446, WO 2012019132 or WO 2015077602.

### <Example 10> Preparation of 2-[(2-methoxy-4-[[4-(4-methylpiperazin-1-yl)piperidin-1-yl]carbonyl]phenyl)amino]-5,11-dimethyl-5,11-dihydro-6H-pyrimido[4,5-b][1,4]benzodiazepin-6-one

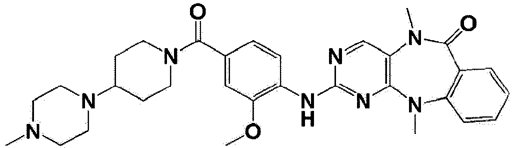

The above compound was prepared with reference to WO 2010080712 or WO 2015176010.

### <Example 11> Preparation of (4-(4-(ethylamino)-5-(trifluoromethyl)pyrimidin-2-ylamino)-2-fluoro-5-methoxyphenyl)(morpholino)methanone

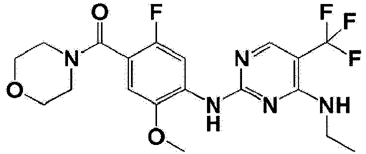

The above compound was prepared with reference to WO 2011151360.

### <Example 12> Preparation of (3-methoxy-4-(4-(methylamino)-5-(trifluoromethyl)pyrimidin-2-ylamino)phenyl)(morpholino)methanone

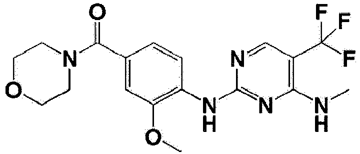

### 12-1. Preparation-1 of (3-methoxy-4-(4-(methylamino)-5-(trifluoromethyl)pyrimidin-2-ylamino)phenyl)(morpholino)methanone

The above compound was prepared with reference to WO 2011151360.

### 12-2. Preparation-2 of (3-methoxy-4-(4-(methylamino)-5-(trifluoromethyl)pyrimidin-2-ylamino)phenyl)(morpholino)methanone

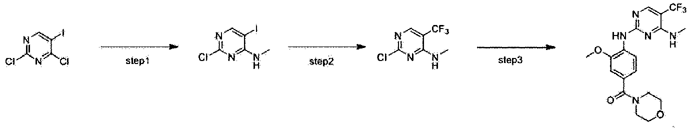

Step 1. 2,4-dichloro-5-iodopyrimidine (1.0 equivalent) was dissolved in THF and then added with methylamine (3.5 wt% in EtOH, 1.1 equivalents) at 0°C. The mixture was stirred at 0°C for 2 hr, the solvent was removed therefrom, and the resulting product was used in the subsequent step without further purification (yield: 100%).

Step 2. A two-necked round-bottom flask was purged with nitrogen gas, and CuI (5.0 equivalents) and KF (5.0 equivalents) were placed therein. The resulting mixture was heated to 150°C and then stirred for 2 hr under reduced pressure. After the reaction, the temperature was decreased to room temperature and trimethyl(trifluoromethyl)silane (5.0 equivalents) dissolved in DMF/NMP (1:1) under nitrogen was added using a syringe. After reaction for 30 min, 2-chloro-5-iodo-N-methylpyrimidine-4-amine (1.0 equivalent) dissolved in DMF/NMP (1:1) was added using a syringe and allowed to react at 50°C for 18 hr. After the reaction, the resulting product was added with water to form a precipitate, which was then filtered off. From the filtrate thus obtained, the organic material was extracted using EtOAc (x3). The combined organic layer was washed with brine, and the remaining water was removed using Na₂SO₄. The dewatered mixture was purified using MPCL (EtOAc:Hex), thereby obtaining a yellow solid target compound (yield: 70%) .

Step 3. 2-chloro-N-methyl-5-(trifluoromethyl)pyrimidine-4-amine (1.0 equivalent) and (4-amino-3-methoxyphenyl)(morpholino)methanone (1.0 equivalent) were dissolved in dioxane and added with p-toluenesulfonic acid (1.0 equivalent) at room temperature. The resulting mixture was stirred at 100°C for 16 hr and cooled to room temperature, the solvent was removed therefrom, and then ice water was added thereto. The organic material was extracted using EtOAc (x3). The combined organic layer was washed with brine, and the remaining water was removed using Na₂SO₄. The dewatered mixture was purified using MPCL (EtOAc:Hex), thereby obtaining a white solid target compound (yield: 70%) .

### <Example 20> Preparation of (2S,6R)-2,6-dimethyl-4-(6-(5-(1-methylcyclopropoxy)-1H-indazol-3-yl)pyrimidin-4-yl)morpholine

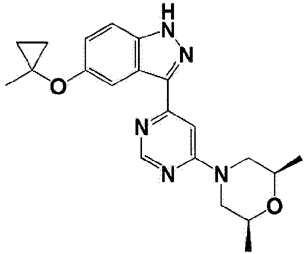

The above compound was prepared with reference to WO 2014137723.

### <Example 21> Preparation of 3-(4-morpholino-1H-pyrrolo[2,3-b]pyridin-3-yl)benzonitrile

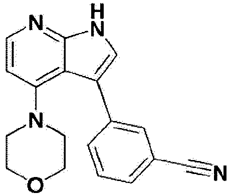

The above compound was prepared with reference to WO 2015092592.

### <Example 22> Preparation of 1-methyl-4-(4-morpholino-7H-pyrrolo[2,3-d]pyrimidin-5-yl)-1H-pyrrole-2-carbonitrile

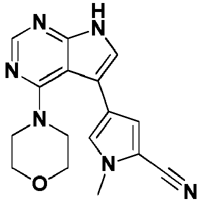

The above compound was prepared with reference to USA 20140005183 A1.

The structures of the compounds prepared in Examples 1 to 22 are shown in Table 1 below.

**[Table 1]**

| Example | Chemical structure | Example | Chemical structure |
|---|---|---|---|
| 1 | 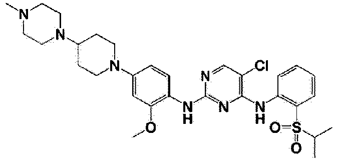 | 12 | 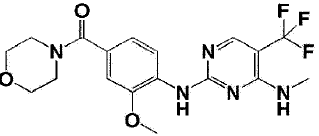 |
| | | 20 | 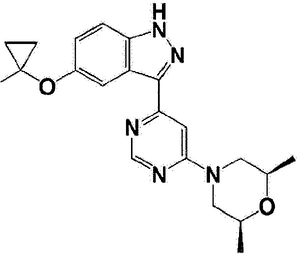 |
| 10 | 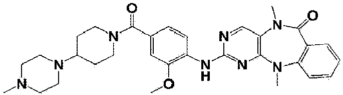 | 21 | 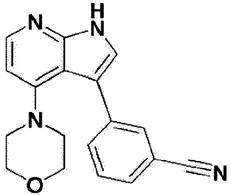 |
| 11 | 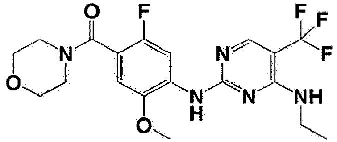 | 22 | 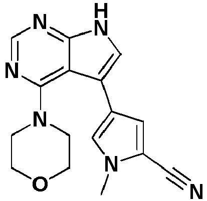 |

### <Comparative Example> Preparation of 2-methyl-2-[3-methyl-4-[[4-(methylamino)-5-(trifluoromethyl)pyrimidin-2-yl]amino]pyrazol-1-yl]propanenitrile

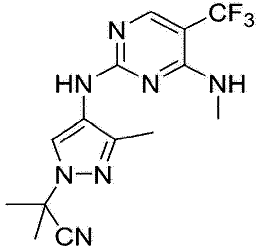

The title compound was prepared with reference to WO 2012062783.

### <Experimental Example 1> Evaluation of LRRK2 protein expression in cancer stem cells of brain tumor patients

In order to evaluate the potential to use the LRRK2 protein as a target protein for the treatment of brain tumors, Western blotting was performed using cancer tissues obtained from 19 brain tumor patients, and LRRK2 protein expression was thus assayed.

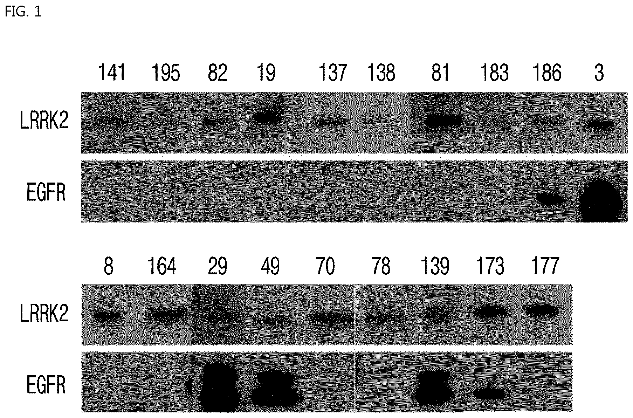

Specifically, cancer tissues were obtained from brain tumor patients, and Western blotting was performed to assay the LRRK2 protein expression. The results are shown in FIG. 1.

As shown in FIG. 1, the LRRK2 protein was expressed in cancer tissues obtained from brain tumor patients, and the expression level was elevated with an increase in brain tumor malignancy. Therefore, it was confirmed that the LRRK2 protein is useful as a target for the treatment of brain tumors.

### <Experimental Example 2> Evaluation of prognosis in brain tumor patients due to LRRK2 protein phosphorylation

The LRRK2 protein exhibits the activity thereof by being phosphorylated. Thus, changes in the prognosis of brain tumor patients due to LRRK2 protein phosphorylation in brain tumor cells were measured.

Specifically, tissue samples were obtained from about 200 stage 3 and stage 4 brain tumor patients, and the expression of phosphorylated LRRK2 protein in the obtained tissue samples was measured through immunostaining.

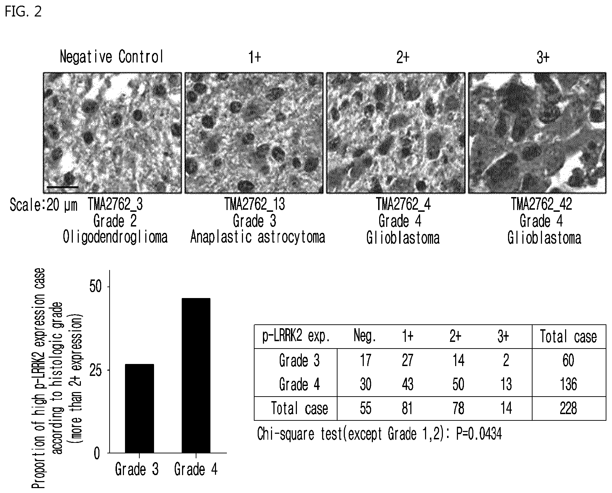

As shown in FIG. 2, the LRRK2 protein phosphorylation increased with an increase in brain tumor malignancy, that is, with progression of the brain tumor from the third stage to the fourth stage. Therefore, it was confirmed that phosphorylated LRRK2 protein is useful as a target protein for the treatment of brain tumors with high malignancy.

### <Experimental Example 3> Correlation between LRRK2 protein mutation and brain tumor

According to conventional studies, it has been reported that various carcinomas may be generated by mutation occurring in the LRRK2 protein, and that Parkinson's disease, which is known to be associated with the LRRK2 protein, is also caused by LRRK2 protein mutation. Thus, whether the LRRK2 protein, which was confirmed to be overexpressed in brain tumor cells in the above Experimental Example, is a mutant thereof was evaluated. Specifically, sequencing was performed using brain tumor cells obtained from five patients using the primers shown in Table 2 below.

**[Table 2]**

| Name | Sequence (5'→3') | SEQ ID NO: |
|---|---|---|
| LRRK2_F1_For | Gagcagcggacgttcatg | SEQ ID NO: 1 |
| LRRK2_F1_rev | Tatgagctgggaaatggcca | SEQ ID NO: 2 |
| LRRK2_F1_For_2 | Gcgggttggaagcaggtg | SEQ ID NO: 3 |
| LRRK2_F1_rev_2 | Tcagcatggagagcatcact | SEQ ID NO: 4 |
| LRRK2_F2_For | Agtacccagagaatgcagca | SEQ ID NO: 5 |
| LRRK2_F2_rev | Gctcccaatagaagcaagca | SEQ ID NO: 6 |
| LRRK2_F3_For | Gaactggacatctgctggc | SEQ ID NO: 7 |
| LRRK2_F3_rev | Ctcgtgagtgcattctggtg | SEQ ID NO: 8 |
| LRRK2_F3_For_2 | Cataggaactggacatctgc | SEQ ID NO: 9 |
| LRRK2_F3_rev_2 | Cattctggtgaagctccagc | SEQ ID NO: 10 |
| LRRK2_F4_For | Ctcaggcagcgatggaaatt | SEQ ID NO: 11 |
| LRRK2_F4_rev | Gacagccaatctcaggagga | SEQ ID NO: 12 |
| LRRK2_F5_For | Gctatgcctttcttgcctcc | SEQ ID NO: 13 |
| LRRK2_F5_rev | Gcagtgctgggtcttgaaaa | SEQ ID NO: 14 |
| LRRK2_F6_For | Cctgtgattctcgttggcac | SEQ ID NO: 15 |
| LRRK2_F6_rev | Aggctgctcggtaaactgat | SEQ ID NO: 16 |
| LRRK2_F7_For | Ttcctgggttgctggagatt | SEQ ID NO: 17 |
| LRRK2_F7_rev | Tcccagacacaatccagctt | SEQ ID NO: 18 |
| LRRK2_F8_For | Acttctgcccaggtctttga | SEQ ID NO: 19 |
| LRRK2_F8_rev | Tgtgagtaccctttccatgtga | SEQ ID NO: 20 |
| LRRK2_F1_500F | Gatctcctcctaacttcag | SEQ ID NO: 21 |
| LRRK2_F1_700r | Caatgcagcacatgaagc | SEQ ID NO: 22 |
| LRRK2_F2_500F | Gaagtggctgaaagtggctg | SEQ ID NO: 23 |
| LRRK2_F2_700r | Gaatatcattcttgaaacac | SEQ ID NO: 24 |
| LRRK2_F3_500F | Catcagcttgctcttaagg | SEQ ID NO: 25 |
| LRRK2_F3_700r | Gaggctgttccttcttcc | SEQ ID NO: 26 |
| LRRK2_F5_500F | Cttataaccgaatgaaac | SEQ ID NO: 27 |
| LRRK2_F5_700r | Ctatagaattcctcacgac | SEQ ID NO: 28 |
| LRRK2_F7_500F | Ggatcgcctgcttcagcag | SEQ ID NO: 29 |
| LRRK2_F7_700r | Cattctacagcagtactg | SEQ ID NO: 30 |
| LRRK2_F8_500F | Gctgctcctttgaagatac | SEQ ID NO: 31 |
| LRRK2_F8_700r | Gtctaccaccactgttatg | SEQ ID NO: 32 |

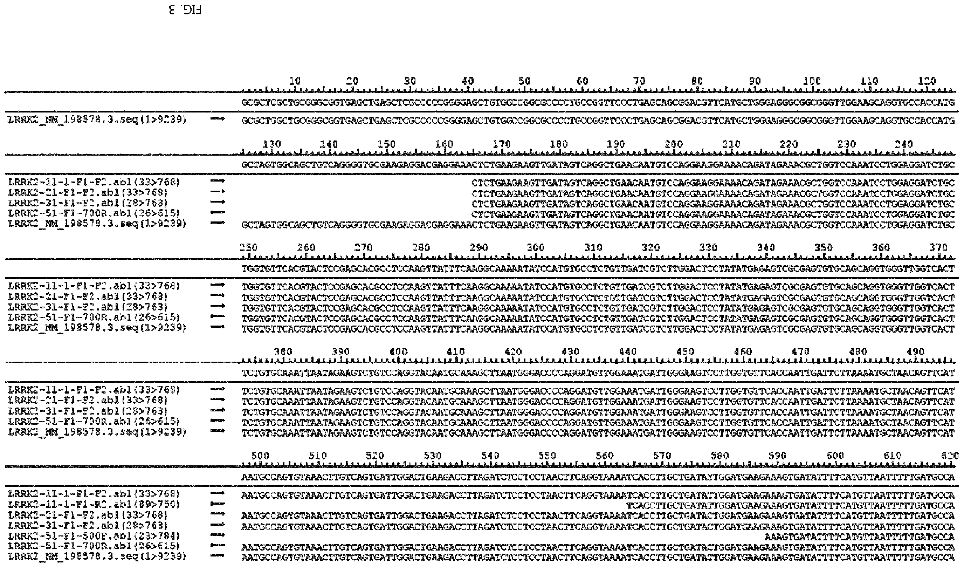

As shown in FIG. 3, nucleic acid sequences encoding the LRRK2 protein expressed in brain tumor cells had no specific mutations such as those commonly found in Parkinson's disease, except for the known single nucleotide polymorphism (SNP). Therefore, it was confirmed that, unlike Parkinson's disease or other types of cancer, brain tumors were caused by overexpressing wild-type LRRK2 protein, resulting in cancer.

### <Experimental Example 4> Evaluation of tumorigenicity due to overexpression of LRRK2 protein

In order to evaluate whether brain tumors were caused by LRRK2 protein overexpression as confirmed in Experimental Example 3, a lentiviral vector (pLenti CMV GFP DEST(736-1), Addgene #19732) containing a polynucleotide encoding the LRRK2 protein was manufactured, and 528NS, 464T (Samsung Seoul Hospital, Korea) and *ex-vivo* cell lines were infected therewith to form tumors. Here, the cells were infected with a lentiviral vector not expressing the LRRK2 protein, as a control.

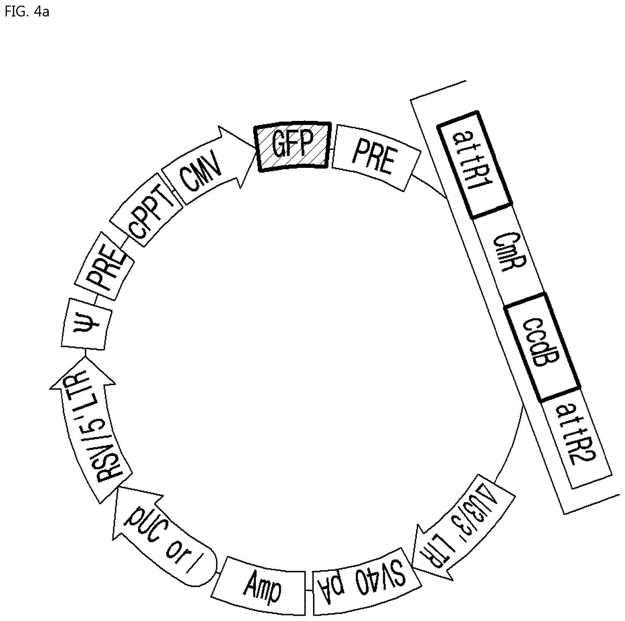

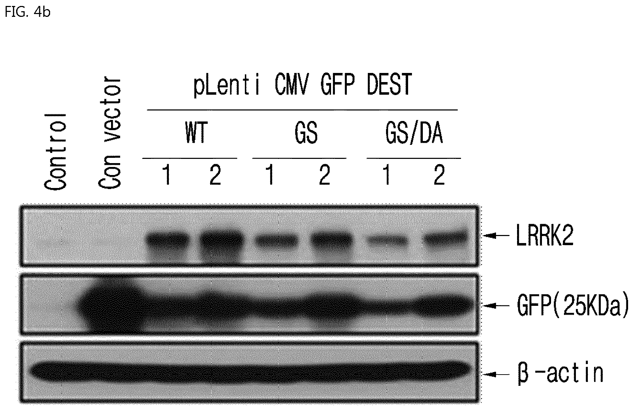

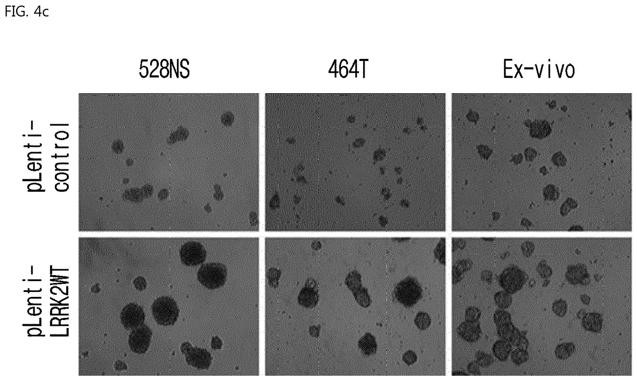

As shown in the results of FIG. 4, the size of GSC (glioma stem cell) spheres was increased in 528NS, 464T or *ex-vivo* cell lines infected with lentivirus expressing the LRRK2 protein compared to the control. Therefore, it was confirmed that the LRRK2 protein overexpression resulted in a tumor.

### <Experimental Example 5> Evaluation of LRRK2 protein phosphorylation inhibitory activity of compound (in vitro)-(1)

In order to evaluate drug efficacy *in vitro,* a Western blotting experiment was performed using brain-tumor-patient-derived cell line NCC01 cells.

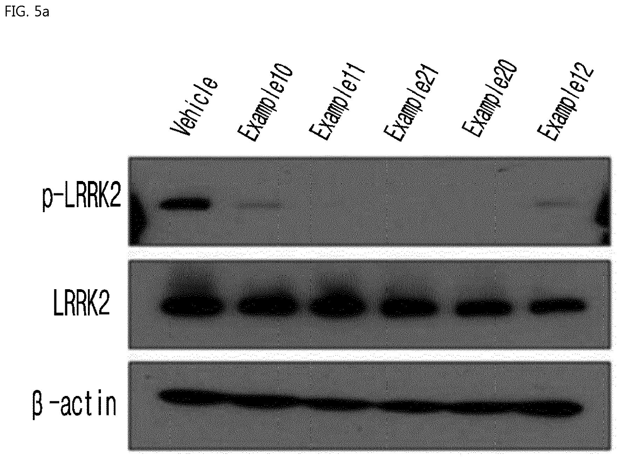

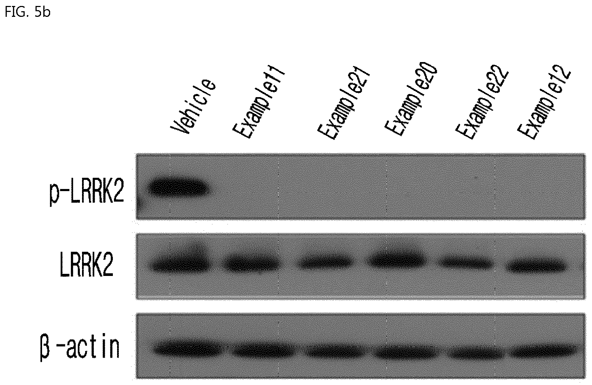

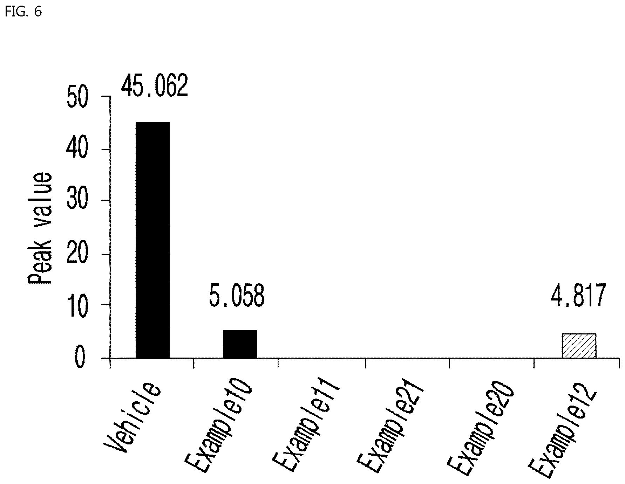

More specifically, 7.5x10⁵ to 1.0x10⁶ NCC01 cells were treated with the compound of each of Examples 10, 11, 12, 20, 21 and 22 at a concentration of 100 or 1,000 nM, and after 1 day, samples were collected and evaluated for LRRK2 protein phosphorylation inhibitory activity via Western blotting. Here, the group not treated with the compound of Example was used as a vehicle. The results are shown in FIGS. 5 and 6.

As shown in FIGS. 5 and 6, the compounds of Examples 10, 11, 12, 20, 21 and 22 according to the present invention significantly inhibited LRRK2 protein phosphorylation in brain-tumor-patient-derived cell line NCC01 cells, compared to the vehicle.

Therefore, it was confirmed that the compound of the present invention inhibited LRRK2 protein phosphorylation expressed in the brain-tumor-patient-derived cell line.

### <Experimental Example 6> Evaluation of LRRK2 protein phosphorylation inhibitory activity of compound depending on compound concentration (in vitro)-(1)

In order to specifically determine the concentration of the compound of Example according to the invention at which high efficacy is exhibited, this experiment was performed in a manner similar to the evaluation of LRRK2 protein phosphorylation inhibitory activity in Experimental Example 1.

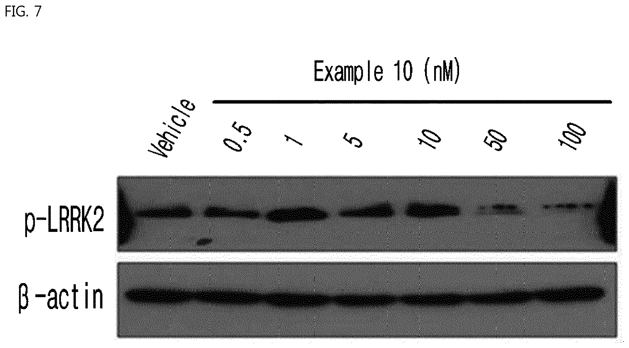

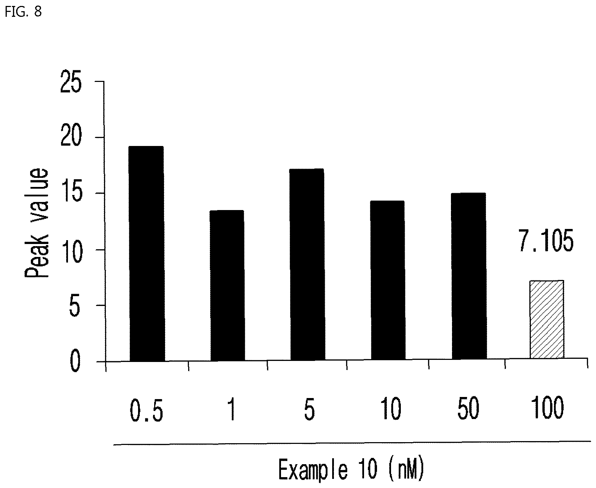

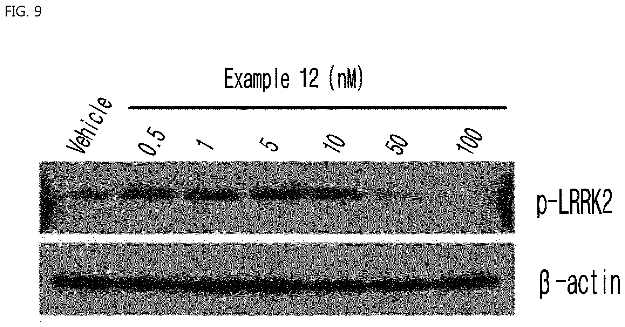

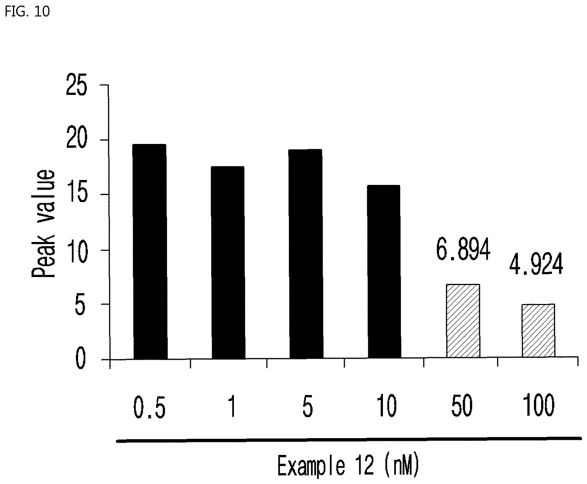

More specifically, 7.5x10⁵ to 1.0x10⁶ NCC01 cells were treated with the compound of each of Examples 10 and 12 at concentrations of 0.5, 1, 5, 10, 50 and 100 nM, and after 1 day, samples were collected and evaluated for LRRK2 protein phosphorylation inhibitory activity via Western blotting. Here, the group not treated with the compound of Example was used as a vehicle. The results are shown in FIGS. 7 to 10.

As shown in FIGS. 7 to 10, the phosphorylated LRRK2 protein was detected when using the compound of Example 10 at a concentration of 100 nM, but the compound of Example 12 mostly inhibited LRRK2 protein phosphorylation at concentrations of 50 and 100 nM.

Therefore, it was confirmed that the compound of the present invention significantly inhibited LRRK2 protein phosphorylation even at low nM concentrations.

### <Experimental Example 7> Evaluation of LRRK2 protein phosphorylation inhibitory activity of compound depending on treatment time (in vitro)

In order to evaluate changes in the activity of the compound of Example according to the present invention over time after treatment therewith, this experiment was performed in a manner similar to the evaluation of LRRK2 protein phosphorylation inhibitory activity in Experimental Example 1.

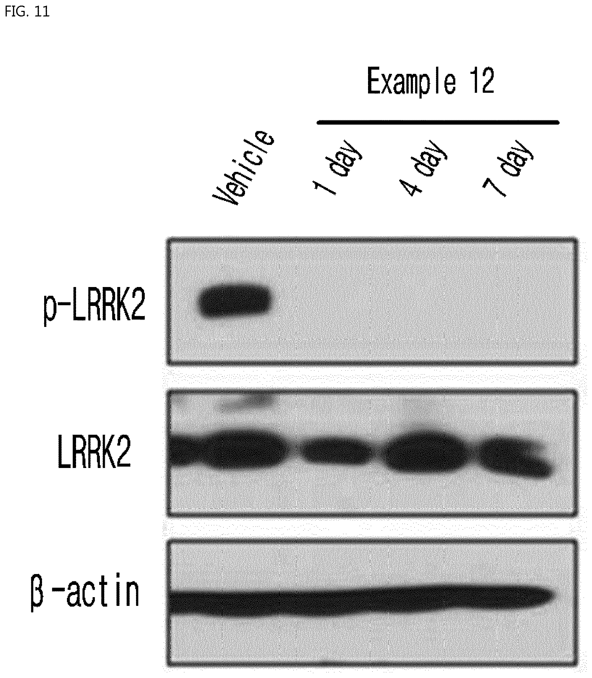

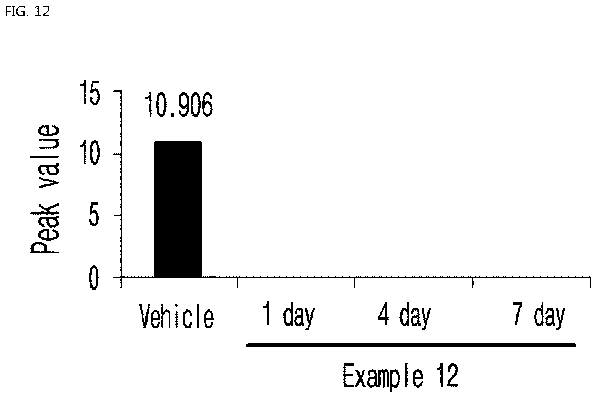

More specifically, 7.5x10⁵ to 1.0x10⁶ NCC01 cells were treated with the compound of Example 12 at a concentration of 100 nM, and after 1 day, 4 days and 8 days, samples were collected and evaluated for LRRK2 protein phosphorylation inhibitory activity via Western blotting. Here, the group not treated with the compound of Example was used as a vehicle. The results are shown in FIGS. 11 and 12.

As shown in FIGS. 11 and 12, the extent of LRRK2 protein phosphorylation was drastically reduced starting 1 day after treatment of brain-tumor-patient-derived cell line NCC01 cells with the compound of Example 12, and the reduction thereof continued over time.

Therefore, it was confirmed that the compound of Example of the present invention inhibited LRRK2 protein phosphorylation continuously for a long period of time.

### <Experimental Example 8> Evaluation of changes in intracellular signaling pathway due to inhibition of LRRK2 protein phosphorylation (in vitro)

An experiment was conducted in order to determine the effect of the compound of Example according to the present invention on the intracellular signaling pathway for cancer stem cell growth.

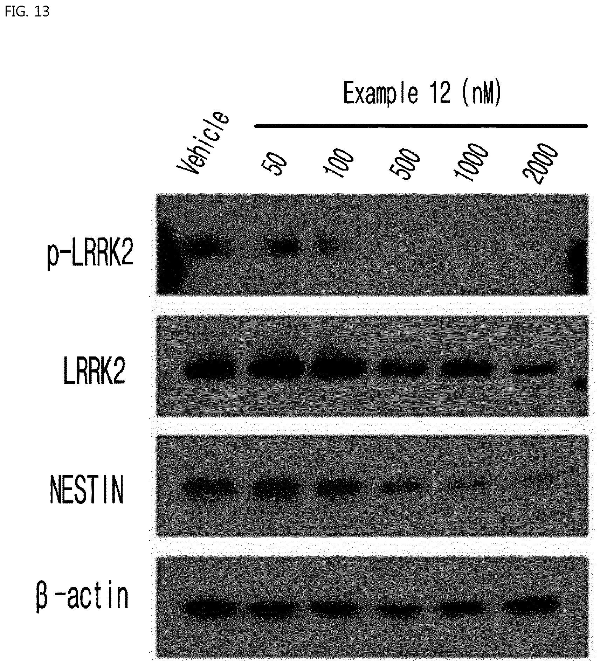

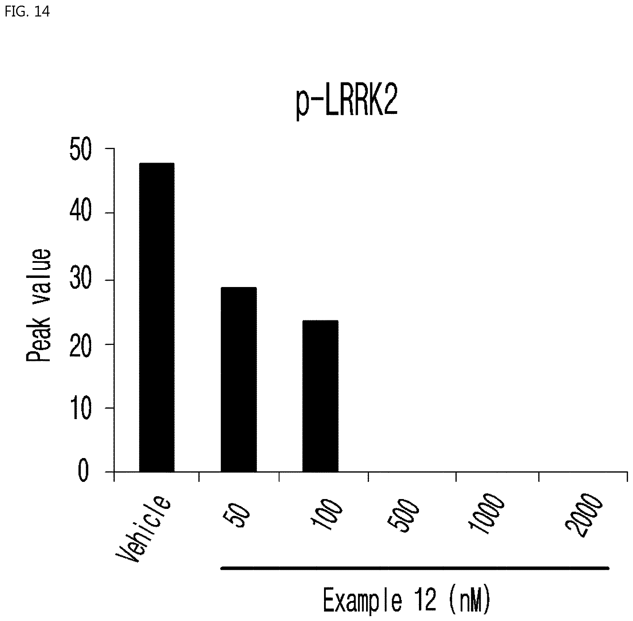

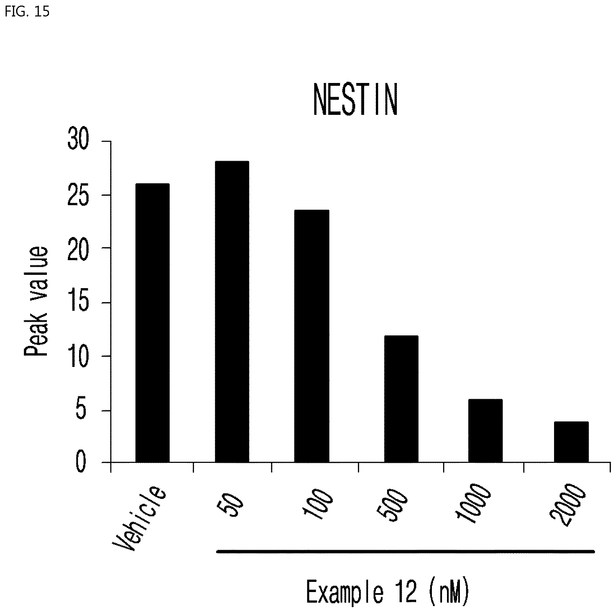

More specifically, 1.0x10⁶ NCC01 cells were treated with the compound of Example 12 at concentrations of 50, 100, 500, 1,000 and 2,000 nM. After 1 day, samples were collected and the expression of NESTIN protein, which is a cancer stem cell marker involved in the intracellular signaling pathway, was measured through Western blotting. Here, the group not treated with the compound of Example was used as a vehicle. The results are shown in FIGS. 13 to 15.

As shown in FIGS. 13 to 15, the LRRK2 protein phosphorylation in NCC01 cells was inhibited by the compound of Example 12, which also decreased in a manner dependent on the concentration of the compound that was used.

Therefore, it was confirmed that the compound of Example 12 according to the present invention inhibited LRRK2-protein-mediated signaling in the cells derived from brain tumor patients, thereby reducing the expression of NESTIN protein involved in tumor stem cell growth and thus inhibiting cell growth.

### <Experimental Example 9> Evaluation of changes in mitochondrial function through inhibition of LRRK2 protein activity

The LRRK2 protein, which is located in the outer membrane of mitochondria, has been reported to be involved in the fragmentation of mitochondria and the regulation of membrane potential thereof. Thus, an experiment was conducted in order to determine the effect of the compound of Example inhibiting the LRRK2 protein on the mitochondrial function.

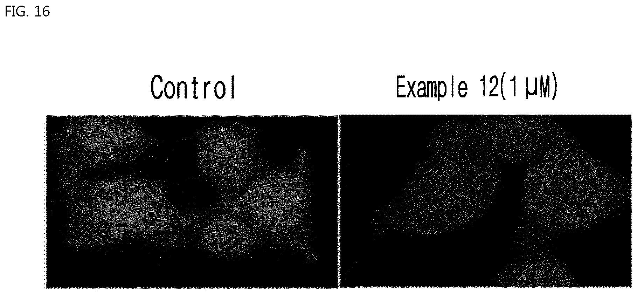

Specifically, NCC01 cells were treated with the compound of Example 12 at 1 µM, and after 1 day, TMRE (tetramethylrhodamine ethylester) dye for staining activated mitochondria was added thereto to evaluate changes in mitochondrial function. Here, the group not treated with the compound of Example was used as a control. The results of staining were observed with a microscope, and are shown in FIG. 16.

As shown in FIG. 16, the activity of the mitochondria in the cells of the control was maintained, whereby the color of the TMRE dye appeared clear. However, in the cells treated with the compound of Example 12, the degree of color development of the TMRE dye was decreased, and moreover, fragmentation of nuclei in the cells occurred. Therefore, it was confirmed that the compound of Example 12 according to the present invention inhibited mitochondrial activity to thus affect the death of brain tumor cells.

### <Experimental Example 10> Evaluation of LRRK2 protein phosphorylation inhibitory activity in brain tumor stem cell line

An experiment was conducted in order to evaluate whether apoptosis of stem cells is induced by inhibiting LRRK2 protein phosphorylation in tumor stem cells, which are known to be associated with tumor metastasis.

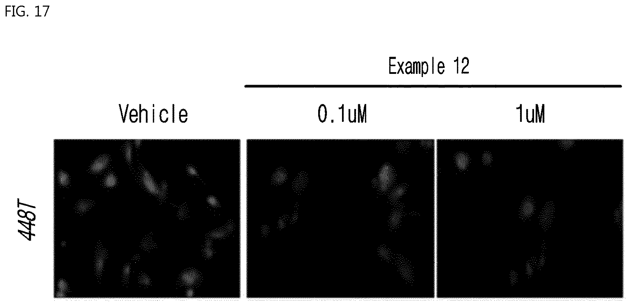

Specifically, brain tumor stem cell line NCC01 cells were treated with the compound of Example 12 at a concentration of 0.1 or 1 µM, and after 1 day, the mitochondria were stained as described in Experimental Example 9 to assay changes in the function thereof. Here, the group not treated with the compound of Example was used as a vehicle. The results of staining were observed with a microscope, and are shown in FIG. 17.

As shown in FIG. 17, the activity of the mitochondria present in the brain tumor stem cells was inhibited in the group treated with the compound of Example 12, compared to the control. Therefore, it was confirmed that the compound according to the present invention inhibited mitochondrial activity to thus induce the death of brain tumor stem cells, resulting in suppressed tumor metastasis.

### <Experimental Example 11> Evaluation of TRAP1 expression inhibitory activity of LRRK2 protein inhibitor in brain tumor stem cell line

An experiment was conducted in order to evaluate whether the LKKR2 protein inhibitor according to the present invention, inhibiting the mitochondrial activity in the brain tumor stem cell line as confirmed in Experimental Example 10, has an influence on changes in expression of TRAP1 (TNF receptor associated protein 1), which is known to be involved in mitochondrial function regulation.

Specifically, brain tumor stem cell line NCC01 cells were added with the compound of Example 12 at a concentration of 100 or 1,000 nM, and after 1 day, changes in the expression of TRAP1 were measured through Western blotting. Here, the group not treated with the compound of Example was used as a vehicle.

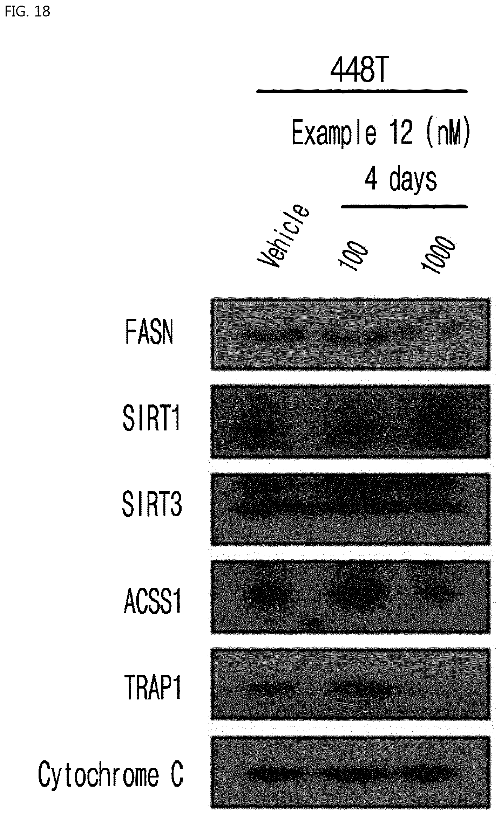

As shown in FIG. 18, the expression of TRAP1 was inhibited by the compound of Example 12. Therefore, it was confirmed that the compound according to the present invention inhibited the expression of TRAP1 to thereby suppress mitochondrial activity.

### <Experimental Example 12> Evaluation of changes in LRRK2 protein expression under hypoxic conditions

Conventional studies have shown that hypoxic conditions are important in the function of GSC. Thus, changes in LRRK2 protein expression under hypoxic conditions and the size of GSC spheres upon inhibiting LRRK2 protein expression were measured.

Specifically, NCC01 and 528NS cells were exposed to hypoxic conditions and treated with two kinds of shRNA against the LRRK2 gene, after which changes in the size of spheres were measured. Here, a control was shRNA (shNT) against luciferase.

**[Table 3]**

| Name | Sequence (5'→3') | SEQ ID NO: |
|---|---|---|
| LRRK2 shRNA-1 | CCGGCCACAAATTCAACGGAAAGAACTCGAGTTCTTTCCG TTGAATTTGTGGTTTTT | SEQ ID NO: 33 |
| LRRK2 shRNA-2 | CCGGCCCAAATTGGTGGAACTCTTACTCGAGTAAGAGTTC CACCAATTTGGGTTTTT | SEQ ID NO: 34 |
| luciferase shRNA | CCGGCGCTGAGTACTTCGAAATGTCCTCGAGGACATTTCG AAGTACTCAGCGTTTTT | SEQ ID NO: 35 |

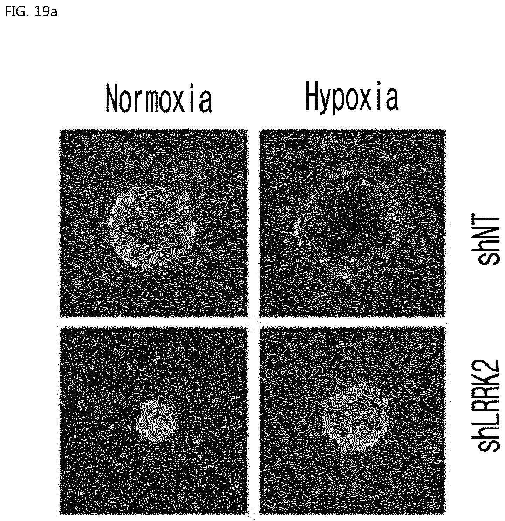

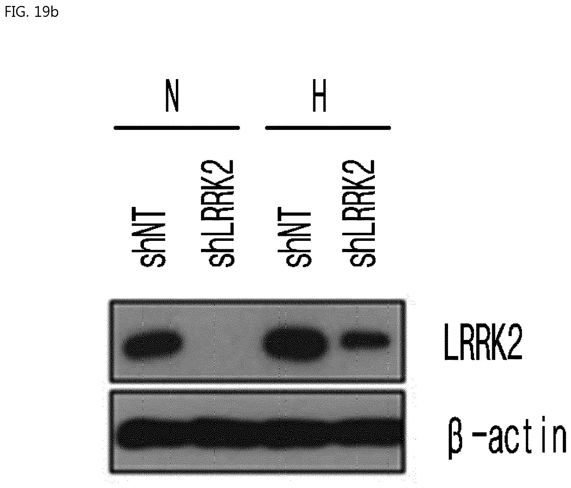

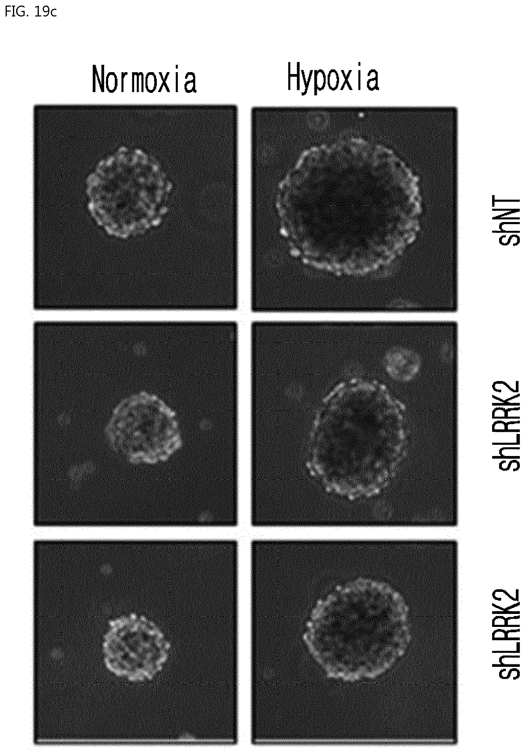

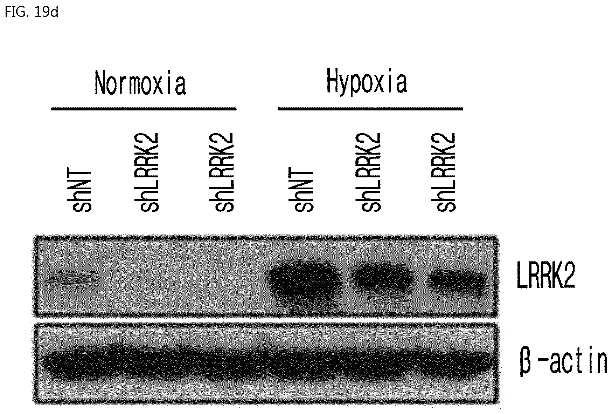

As shown in FIG. 19, the sphere size of the NCC01 (a and b) and 528NS (c and d) cells exposed to hypoxic conditions was increased compared to the control, but was suppressed by LRRK2 shRNA-1 and LRRK2 shRNA-2, which are two kinds of shRNA against the LRRK2 gene. Therefore, it was confirmed that the growth of GSC, produced under hypoxic conditions, was suppressed through inhibition of LRRK2 protein expression.

### <Experimental Example 13> Evaluation of apoptosis induction activity through inhibition of LRRK2 protein phosphorylation (in vitro)

In order to evaluate whether apoptosis of brain tumor cells is induced by inhibiting LRRK2 protein phosphorylation, an experiment was conducted using FACS (fluorescence-activated cell sorting).

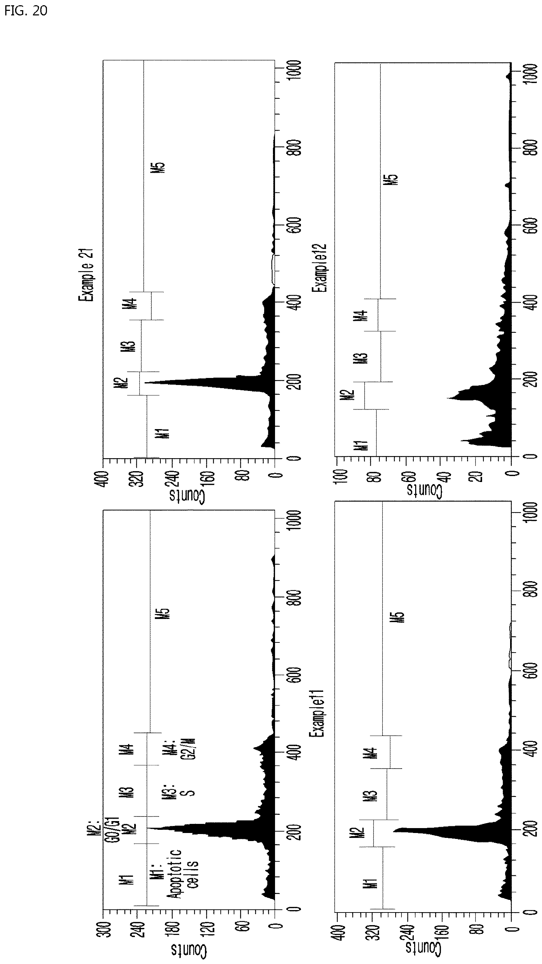

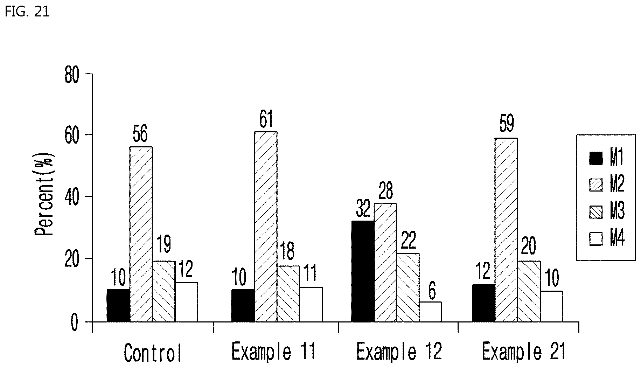

More specifically, 7.5x10⁵ to 1.0x10⁶ NCC01 cells were treated with the compound of each of Examples 11, 12 and 21 at a concentration of 100 nM, and after 1 day, samples were collected and subjected to FACS, thus assaying the apoptosis induction activity. The group not treated with the compound of Example was used as a control. The results are shown in FIGS. 20 and 21.

As shown in FIGS. 20 and 21, the compounds of Examples 11, 12 and 21 induced the apoptosis of NCC01 cells. In particular, the compound of Example 12 exhibited an apoptosis rate about 3 times as high as the compounds of Examples 11 and 21, indicating that the compound of Example 12 according to the present invention was very effective in inducing apoptosis compared to the compounds of other Examples.

### <Experimental Example 14> Evaluation of growth inhibitory activity of compound on brain tumor cells in animal model-(1)

An experiment was conducted in order to evaluate whether the compound of Example according to the present invention exhibits the growth inhibitory activity on brain tumor cells in an animal model.

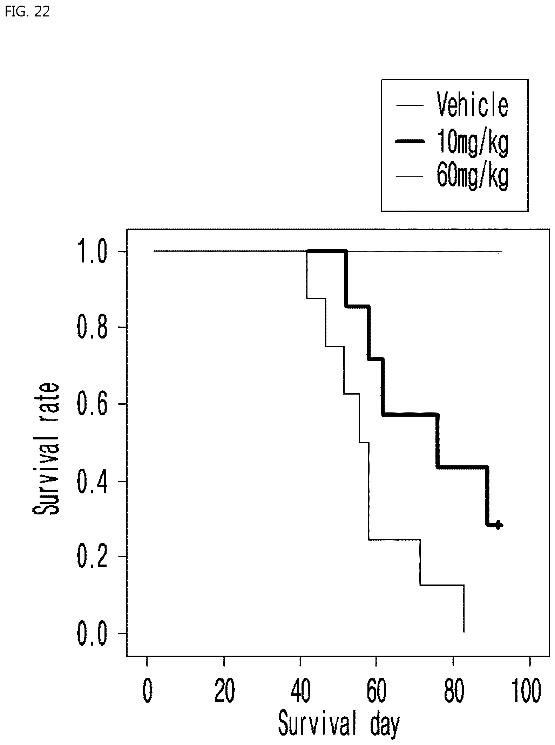

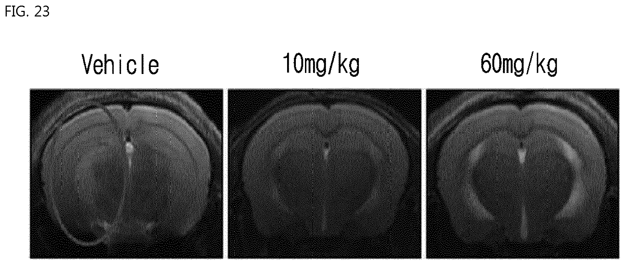

More specifically, 1.0x10⁴ NCC01 cells, a brain-tumor-patient-derived cell line, were injected into the brain of a Balb/c-nude mouse model, and after 3 days, the compound of Example 12 was orally administered at a dose of 10 mg/kg or 60 mg/kg. After administration of the compound, the survival rate of the mice based on the survival period was determined, and the size of the tumors generated in the mice was measured using MRI. Here, the group not treated with the compound of Example was used as a vehicle. The results are shown in FIGS. 22 and 23.

As shown in FIG. 22, the average survival period of the mice was 54 days in the vehicle and was 71 days in the group orally administered with the compound of Example 12 at 10 mg/kg, and all the mice in the group orally administered with the compound of Example 12 at 60 mg/kg survived up to 100 days (Vehicle vs 10 mg/kg: p=0.032, Vehicle vs 60 mg/kg: p<0.001).

As shown in FIG. 23, the tumor was formed at the position to which the NCC01 cells were injected in the vehicle not treated with the compound, but no tumor was observed in the group orally administered with the compound of Example 12 at 10 mg/kg or 60 mg/kg.

Therefore, it was confirmed that the compound of Example according to the present invention significantly inhibited the formation of tumors derived from brain tumor cells in the animal model.

### <Experimental Example 15> Evaluation of therapeutic effect on brain tumor upon co-administration of compound and Avastin

An experiment was conducted in order to determine the therapeutic effect in an animal model when the compound of Example according to the present invention was administered in combination with Avastin, prescribed as a drug for treating brain tumors.

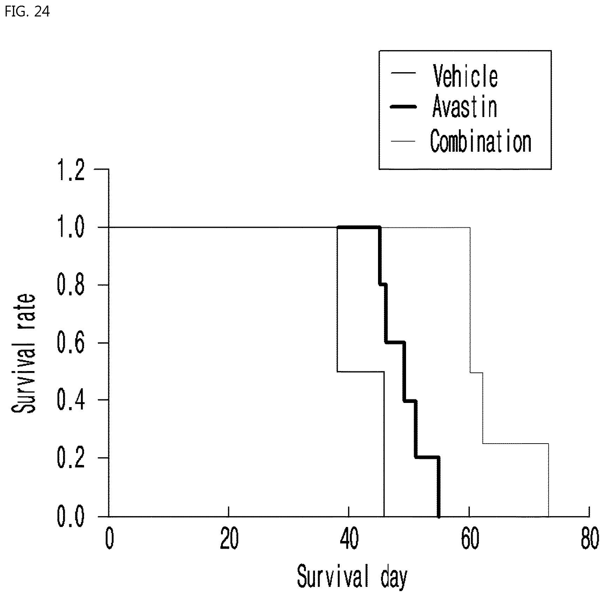

More specifically, orthotopic mouse models were manufactured using 5-week-old Balb/c-nude mice. U87MG cells (ATCC, USA), a brain tumor cell line, were injected 3.5 mm deep at a position 2.2 mm to the left of and 0.5 mm behind the bregma using a stereotaxic device. 3 days after injection of the cells, mice were divided into three groups of 5 mice per group (a control (vehicle), a group administered with Avastin, and a group administered with the compound of Example 12 and Avastin), and the drug was administered thereto. The compound of Example 12 was orally administered at a dose of 30 mg/kg and Avastin was injected intraperitoneally at a dose of 10 mg/kg. Thereafter, the survival period was measured when the mice showed evidence of tumor formation such as a reduction in 20% or more of the total body weight thereof or abnormal behavior. The survival graph obtained using the statistical program is shown in FIG. 24.

As shown in FIG. 24, when the compound of Example 12 was administered in combination with Avastin, the therapeutic effect of Avastin on brain tumors was further significantly increased.

### <Experimental Example 16> Evaluation of LRRK2 protein phosphorylation inhibitory activity of compound of Comparative Example (in vitro)

A Western blotting experiment was carried out using brain-tumor-patient-derived cell line NCC01 cells in order to evaluate *in-vitro* drug efficacy of the compound of Comparative Example, known as an LRRK2 protein inhibitor.

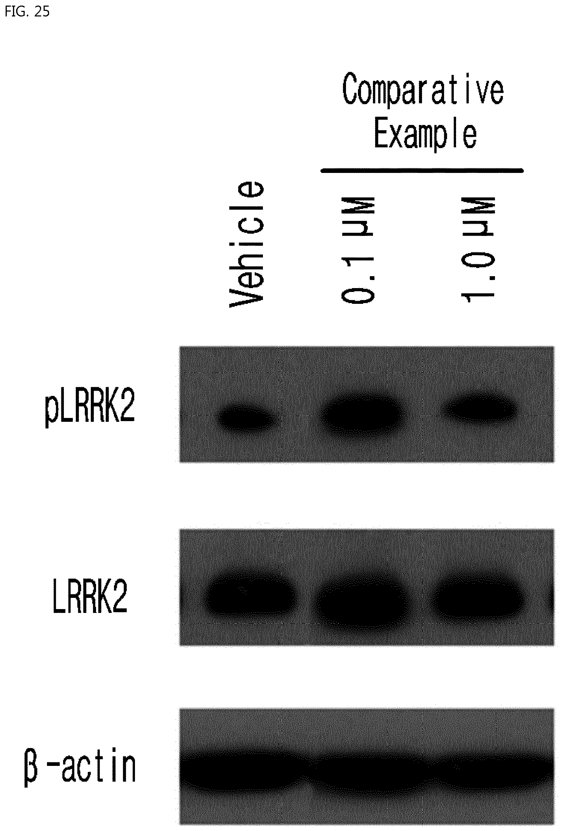

More specifically, 7.5x10⁵ to 1.0x10⁶ cells were treated with the compound of Comparative Example at a concentration of 0.1 or 1.0 µM, and after 1 day, samples were collected and evaluated for LRRK2 protein phosphorylation inhibitory activity through Western blotting. Here, the group not treated with the compound of Example was used as a vehicle. The results are shown in FIG. 25.

As shown in FIG. 25, the compound of Comparative Example according to the present invention was known to inhibit LRRK2 protein expression, but did not inhibit LRRK2 protein phosphorylation.

### <Experimental Example 17> Evaluation of LRRK2 protein phosphorylation inhibitory activity of compound (in vitro)-(2)

A Western blotting experiment was carried out using brain-tumor-patient-derived cell line 448T cells in order to evaluate *in-vitro* drug efficacy. Here, the experiment was conducted in the same manner under the same conditions as in Experimental Example 5, with the exception that 448T cells (Samsung Seoul Hospital, Korea) were used in lieu of NCC01 cells, and the compounds of Examples 10, 11, 12, 20 and 21 were used.

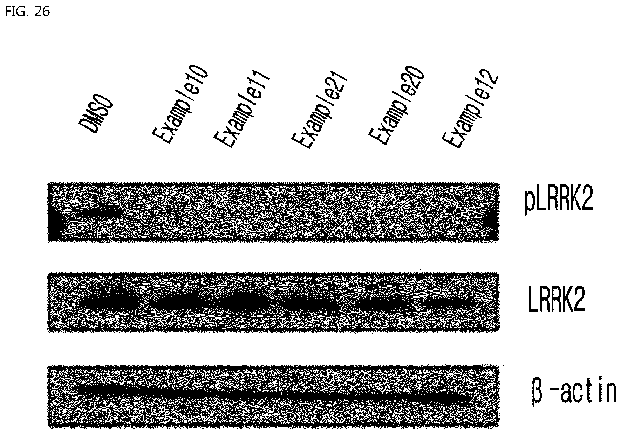

As shown in FIG. 26, the compounds of Examples 10, 11, 12, 20 and 21 according to the present invention significantly inhibited LRRK2 protein phosphorylation in the brain-tumor-patient-derived cell line 448T cells, compared to the vehicle.

Therefore, it was confirmed again that the compound according to the present invention inhibited LRRK2 protein phosphorylation expressed in the brain-tumor-patient-derived cell line.

### <Experimental Example 18> Evaluation of LRRK2 protein phosphorylation inhibitory activity of compound depending on compound concentration (in vitro)-(2)

An experiment was conducted in order to determine the concentration of the compound of Example according to the invention, which is able to exhibit high efficacy in 448T cells. Specifically, the experiment was performed in the same manner under the same conditions as in Experimental Example 6, with the exception that 448T cells were used in lieu of NCC01 cells, and the compound of Example 12 was used at concentrations of 50, 100, 500, 1,000, and 2,000 nM.

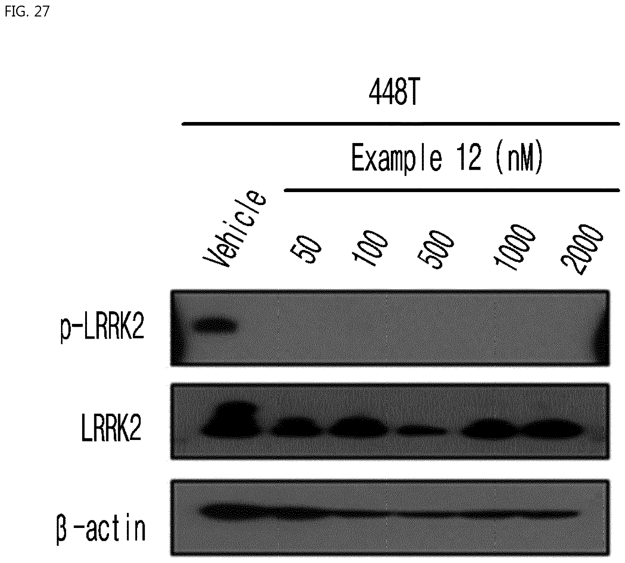

As shown in FIG. 27, the compound of Example 12 inhibited most of the LRRK2 protein phosphorylation at a concentration of 50 nM.

Therefore, it was confirmed again that the compound according to the present invention significantly inhibited LRRK2 protein phosphorylation even at low nM concentrations.

### <Experimental Example 19> Evaluation of growth inhibitory activity of compound on brain tumor cells in animal model-(2)

An experiment was conducted in order to evaluate whether the compound of Example according to the present invention exhibits the growth inhibitory activity on brain tumor cells in an animal model. Specifically, the experiment was conducted in the same manner under the same conditions as in Experimental Example 14, with the exception that 448T cells were used in lieu of NCC01 cells, and the compound of each of Examples 12, 20 and 21 was orally administered at a dose of 60 mg/kg.

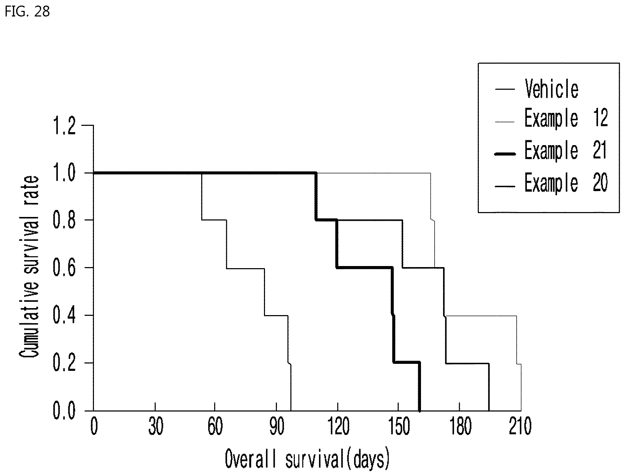

As shown in FIG. 28, the tumor was formed at the position to which the 448T cells were injected in the vehicle not treated with the compound, but no tumor was observed in the group orally administered with the compound of each of Examples 12, 20 and 21.

Therefore, it was confirmed again that the compound of Example according to the present invention significantly inhibited the formation of tumors derived from brain tumor cells in the animal model.

### <Experimental Example 20> Evaluation of LRRK2 protein phosphorylation inhibitory activity of compound of Example and Comparative Example (in vitro)

An experiment was conducted in order to evaluate the *in-vitro* drug efficacy of the compound of Comparative Example, known as an LRRK2 protein inhibitor. Specifically, the experiment was conducted in the same manner under the same conditions as in Experimental Example 16, with the exception that 448T cells were used in lieu of NCC01 cells, and the compounds of Examples 1, 11 and 12 were used.

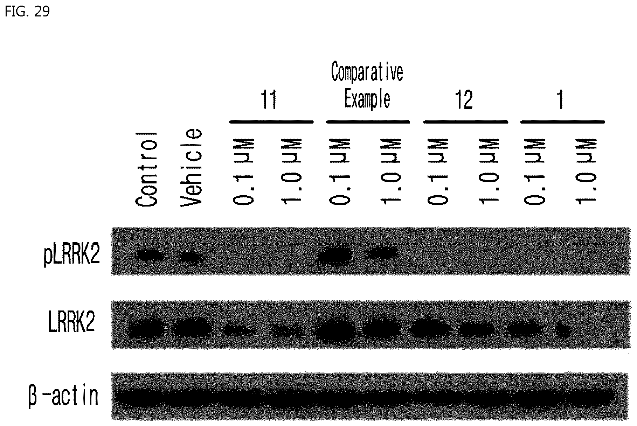

As shown in FIG. 29, the compound of Comparative Example was known to inhibit LRRK2 protein expression, but did not inhibit LRRK2 protein phosphorylation, unlike the compounds of Examples 1, 11 and 12 according to the present invention.

### <Formulation Example 1> Preparation of pharmaceutical formulation

### 1-1. Preparation of powder

500 mg of the LRRK2 protein inhibitor
100 mg of lactose
10 mg of talc
A powder was manufactured by mixing the above components and placing the mixture in an airtight bag.

### 1-2. Preparation of tablet

500 mg of the LRRK2 protein inhibitor
100 mg of corn starch
100 mg of lactose
2 mg of magnesium stearate
A tablet was manufactured by mixing the above components and tableting the mixture in accordance with a typical tablet-manufacturing method.

### 1-3. Preparation of capsule

500 mg of the LRRK2 protein inhibitor
100 mg of corn starch
100 mg of lactose
2 mg of magnesium stearate
A capsule was manufactured by mixing the above components and placing the mixture in a gelatin capsule in accordance with a typical capsule-manufacturing method.

### 1-4. Preparation of injection

500 mg of the LRRK2 protein inhibitor
Appropriate amount of sterile distilled water for injection
Appropriate amount of pH modifier
An injection was manufactured using the above amounts of the components per ampoule (2 ml) in accordance with a typical injection-manufacturing method.

### 1-5. Preparation of liquid

100 mg of the LRRK2 protein inhibitor
10 g of isomerized sugar
5 g of mannitol
Appropriate amount of purified water
A liquid was manufactured by dissolving the above components in purified water, adding an appropriate amount of lemon flavor, mixing the above components, adding purified water such that the total volume was 100 ml, placing the resulting mixture in a brown bottle and performing sterilization, in accordance with a typical liquid-manufacturing method.
## Claims

1. A pharmaceutical composition for use in preventing or treating brain cancer, containing an LRRK2 (leucine-rich repeat kinase-2) protein inhibitor selected from the following compound group as an active ingredient:
(1) 5-chloro-N4-(2-(isopropylsulfonyl)phenyl)-N2-(2-methoxy-4-(4-(4-methylpiperazin-1-yl)piperidin-1-yl)phenyl)pyrimidine-2,4-diamine; (10) 2-[(2-methoxy-4-[[4-(4-methylpiperazin-1-yl)piperidin-1-yl]carbonyl]phenyl)amino]-5,11-dimethyl-5,11-dihydro-6H-pyrimido[4,5-b][1,4]benzodiazepin-6-one; (11) (4-(4-(ethylamino)-5-(trifluoromethyl)pyrimidin-2-ylamino)-2-fluoro-5-methoxyphenyl)(morpholino)methanone; (12) (3-methoxy-4-(4-(methylamino)-5-(trifluoromethyl)pyrimidin-2-ylamino)phenyl)(morpholino)methanone; (20) (2S,6R)-2,6-dimethyl-4-(6-(5-(1-methylcyclopropoxy)-1H-indazol-3-yl)pyrimidin-4-yl)morpholine; (21) 3-(4-morpholino-1H-pyrrolo[2,3-b]pyridin-3-yl)benzonitrile; and (22) 1-methyl-4-(4-morpholino-7H-pyrrolo[2,3-d]pyrimidin-5-yl)-1H-pyrrole-2-carbonitrile.

2. A pharmaceutical kit for use in preventing or treating brain cancer, comprising:
a first component containing a pharmaceutically effective amount of an LRRK2 protein inhibitor selected from the following compound group as an active ingredient;
(1) 5-chloro-N4-(2-(isopropylsulfonyl)phenyl)-N2-(2-methoxy-4-(4-(4-methylpiperazin-1-yl)piperidin-1-yl)phenyl)pyrimidine-2,4-diamine; (10) 2-[(2-methoxy-4-[[4-(4-methylpiperazin-1-yl)piperidin-1-yl]carbonyl]phenyl)amino]-5,11-dimethyl-5,11-dihydro-6H-pyrimido[4,5-b][1,4]benzodiazepin-6-one; (11) (4-(4-(ethylamino)-5-(trifluoromethyl)pyrimidin-2-ylamino)-2-fluoro-5-methoxyphenyl)(morpholino)methanone; (12) (3-methoxy-4-(4-(methylamino)-5-(trifluoromethyl)pyrimidin-2-ylamino)phenyl)(morpholino)methanone; (20) (2S,6R)-2,6-dimethyl-4-(6-(5-(1-methylcyclopropoxy)-1H-indazol-3-yl)pyrimidin-4-yl)morpholine; (21) 3-(4-morpholino-1H-pyrrolo[2,3-b]pyridin-3-yl)benzonitrile; and (22) 1-methyl-4-(4-morpholino-7H-pyrrolo[2,3-d]pyrimidin-5-yl)-1H-pyrrole-2-carbonitrile; and
a second component containing a pharmaceutically effective amount of Avastin® (bevacizumab) as an active ingredient.

3. The pharmaceutical kit for use of claim 2, wherein the first component and the second component are sequentially or simultaneously administered.

## Patentansprüche

1. Pharmazeutische Zusammensetzung zur Verwendung bei der Prävention oder Behandlung von Hirnkrebs, enthaltend einen LRRK2-Protein-Inhibitor (Leucinreiche-Repeat-Kinase-2-Protein-Inhibitor), der aus der folgenden Verbindungsgruppe ausgewählt ist, als einen Wirkstoff:
(1) 5-Chlor-N4-(2-(isopropylsulfonyl)phenyl)-N2-(2-methoxy-4-(4-(4-methylpiperazin-1-yl)piperidin-1-yl)phenyl)pyrimidin-2,4-diamin; (10) 2-[(2-Methoxy-4-[[4-(4-methylpiperazin-1-yl)piperidin-1-yl]carbonyl]phenyl)amino]-5,11-dimethyl-5,11-dihydro-6H-pyrimido[4,5-b][1,4]benzodiazepin-6-on; (11) (4-(4-(Ethylamino)-5-(trifluormethyl)pyrimidin-2-ylamino)-2-fluor-5-methoxyphenyl)(morpholino)methanon; (12) (3-Methoxy-4-(4-(methylamino)-5-(trifluormethyl)pyrimidin-2-ylamino)phenyl)(morpholino)methanon; (20) (2S,6R)-2,6-Dimethyl-4-(6-(5-(1-methylcyclopropoxy)-1H-indazol-3-yl)pyrimidin-4-yl)morpholin; (21) 3-(4-Morpholino-1H-pyrrolo[2,3-b]pyridin-3-yl)benzonitril und (22) 1-Methyl-4-(4-morpholino-7H-pyrrolo[2,3-d]pyrimidin-5-yl)-1H-pyrrol-2-carbonitril.

2. Pharmazeutisches Kit zur Verwendung bei der Prävention oder Behandlung von Hirnkrebs, umfassend:
eine erste Komponente, enthaltend eine pharmazeutisch wirksame Menge eines LRRK2-Protein-Inhibitors, der aus der folgenden Verbindungsgruppe ausgewählt ist, als einen Wirkstoff:
(1) 5-Chlor-N4-(2-(isopropylsulfonyl)phenyl)-N2-(2-methoxy-4-(4-(4-methylpiperazin-1-yl)piperidin-1-yl)phenyl)pyrimidin-2,4-diamin; (10) 2-[(2-Methoxy-4-[[4-(4-methylpiperazin-1-yl)piperidin-1-yl]carbonyl]phenyl)amino]-5,11-dimethyl-5,11-dihydro-6H-pyrimido[4,5-b][1,4]benzodiazepin-6-on; (11) (4-(4-(Ethylamino)-5-(trifluormethyl)pyrimidin-2-ylamino)-2-fluor-5-methoxyphenyl)(morpholino)methanon; (12) (3-Methoxy-4-(4-(methylamino)-5-(trifluormethyl)pyrimidin-2-ylamino)phenyl)(morpholino)methanon; (20) (2S,6R)-2,6-Dimethyl-4-(6-(5-(1-methylcyclopropoxy)-1H-indazol-3-yl)pyrimidin-4-yl)morpholin; (21) 3-(4-Morpholino-1H-pyrrolo[2,3-b]pyridin-3-yl)benzonitril und (22) 1-Methyl-4-(4-morpholino-7H-pyrrolo[2,3-d]pyrimidin-5-yl)-1H-pyrrol-2-carbonitril; und
eine zweite Komponente, enthaltend eine pharmazeutisch wirksame Menge von Avastin® (Bevacizumab) als einen Wirkstoff.

3. Pharmazeutisches Kit zur Verwendung nach Anspruch 2, wobei die erste Komponente und die zweite Komponente nacheinander oder gleichzeitig verabreicht werden.

## Revendications

1. Composition pharmaceutique pour l'utilisation dans la prévention ou le traitement du cancer du cerveau, contenant un inhibiteur de protéine LRRK2 (kinase-2 à répétition riche en leucine) sélectionné parmi le groupe des composés suivants comme ingrédient actif :
(1) 5-chloro-N4-(2-(isopropylsulfonyl)phényl)-N2-(2-méthoxy-4-(4-(4-méthylpipérazin-1-yl)pipéridin-1-yl)phényl)pyrimidine-2,4-diamine ; (10) 2-[(2-méthoxy-4-[[4-(4-méthylpipérazin-1-yl)pipéridin-1-yl]carbonyl]phényl)amino]-5,11-diméthyl-5,11-dihydro-6H-pyrimido[4,5-b][1,4]benzodiazépin-6-one ; (11) (4-(4-(éthylamino)-5-(trifluorométhyl)pyrimidin-2-ylamino)-2-fluoro-5-méthoxyphényl)(morpholino)méthanone ; (12) (3-méthoxy-4-(4-(méthylamino)-5-(trifluorométhyl)pyrimidin-2-ylamino)phényl)(morpholino)méthanone ; (20) (2S,6R)-2,6-diméthyl-4-(6-(5-(1-méthylcyclopropoxy)-1H-indazol-3-yl)pyrimidin-4-yl)morpholine ; (21) 3-(4-morpholino-1H-pyrrolo[2,3-b]pyridin-3-yl)benzonitrile ; et (22) 1-méthyl-4-(4-morpholino-7H-pyrrolo[2,3-d]pyrimidin-5-yl)-1H-pyrrole-2-carbonitrile.

2. Kit pharmaceutique pour l'utilisation dans la prévention ou le traitement du cancer du cerveau, comprenant :
un premier composant contenant une quantité pharmaceutiquement efficace d'un inhibiteur de protéine LRRK2 sélectionné parmi le groupe des composés suivants comme ingrédient actif :
(1) 5-chloro-N4-(2-(isopropylsulfonyl)phényl)-N2-(2-méthoxy-4-(4-(4-méthylpipérazin-1-yl)pipéridin-1-yl)phényl)pyrimidine-2,4-diamine ; (10) 2-[(2-méthoxy-4-[[4-(4-méthylpipérazin-1-yl)pipéridin-1-yl]carbonyl]phényl)amino]-5,11-diméthyl-5,11-dihydro-6H-pyrimido[4,5-b][1,4]benzodiazépin-6-one ; (11) (4-(4-(éthylamino)-5-(trifluorométhyl)pyrimidin-2-ylamino)-2-fluoro-5-méthoxyphényl)(morpholino)méthanone ; (12) (3-méthoxy-4-(4-(méthylamino)-5-(trifluorométhyl)pyrimidin-2-ylamino)phényl)(morpholino)méthanone ; (20) (2S,6R)-2,6-diméthyl-4-(6-(5-(1-méthylcyclopropoxy)-1H-indazol-3-yl)pyrimidin-4-yl)morpholine ; (21) 3-(4-morpholino-1H-pyrrolo[2,3-b]pyridin-3-yl)benzonitrile ; et (22) 1-méthyl-4-(4-morpholino-7H-pyrrolo[2,3-d]pyrimidin-5-yl)-1H-pyrrole-2-carbonitrile; et
un second composant contenant une quantité pharmaceutiquement efficace d'Avastin® (bevacizumab) comme ingrédient actif.

3. Kit pharmaceutique pour l'utilisation selon la revendication 2, le premier composant et le second composant étant séquentiellement ou simultanément administrés.


<110> Daegu-Gyeongbuk Medical Innovation Foundation
   NATIONAL CANCER CENTER
   SAMSUNG LIFE PUBLIC WELFARE FOUNDATION
<120> Pharmaceutical composition for preventing or treating brain cancer comprising compounds penetrating blood-brain barrier
<130> 2018FP0-01-010_PCT
<150> KR 10-2017-0024812
   <151> 2017-02-24
<160> 35
<170> KoPatentIn 3.0
<210> 1
   <211> 18
   <212> DNA
   <213> Artificial Sequence
<220>
   <223> LRRK2_F1_For
<400> 1
   gagcagcgga cgttcatg 18
<210> 2
   <211> 20
   <212> DNA
   <213> Artificial Sequence
<220>
   <223> LRRK2_F1_rev
<400> 2
   tatgagctgg gaaatggcca 20
<210> 3
   <211> 18
   <212> DNA
   <213> Artificial Sequence
<220>
   <223> LRRK2_F1_For_2
<400> 3
   gcgggttgga agcaggtg 18
<210> 4
   <211> 20
   <212> DNA
   <213> Artificial Sequence
<220>
   <223> LRRK2_F1_rev_2
<400> 4
   tcagcatgga gagcatcact 20
<210> 5
   <211> 20
   <212> DNA
   <213> Artificial Sequence
<220>
   <223> LRRK2_F2_For
<400> 5
   agtacccaga gaatgcagca 20
<210> 6
   <211> 20
   <212> DNA
   <213> Artificial Sequence
<220>
   <223> LRRK2_F2_rev
<400> 6
   gctcccaata gaagcaagca 20
<210> 7
   <211> 19
   <212> DNA
   <213> Artificial Sequence
<220>
   <223> LRRK2_F3_For
<400> 7
   gaactggaca tctgctggc 19
<210> 8
   <211> 20
   <212> DNA
   <213> Artificial Sequence
<220>
   <223> LKRK2_F3_rev
<400> 8
   ctcgtgagtg cattctggtg 20
<210> 9
   <211> 20
   <212> DNA
   <213> Artificial Sequence
<220>
   <223> LRRK2_F3_For_2
<400> 9
   cataggaact ggacatctgc 20
<210> 10
   <211> 20
   <212> DNA
   <213> Artificial Sequence
<220>
   <223> LKRK2_F3_rev_2
<400> 10
   cattctggtg aagctccagc 20
<210> 11
   <211> 20
   <212> DNA
   <213> Artificial Sequence
<220>
   <223> LRRK2_F4_For
<400> 11
   ctcaggcagc gatggaaatt 20
<210> 12
   <211> 20
   <212> DNA
   <213> Artificial Sequence
<220>
   <223> LRRK2_F4_rev
<400> 12
   gacagccaat ctcaggagga 20
<210> 13
   <211> 20
   <212> DNA
   <213> Artificial Sequence
<220>
   <223> LRRK2_F5_For
<400> 13
   gctatgcctt tcttgcctcc 20
<210> 14
   <211> 20
   <212> DNA
   <213> Artificial Sequence
<220>
   <223> LRRK2_F5_rev
<400> 14
   gcagtgctgg gtcttgaaaa 20
<210> 15
   <211> 20
   <212> DNA
   <213> Artificial Sequence
<220>
   <223> LRRK2_F6_For
<400> 15
   cctgtgattc tcgttggcac 20
<210> 16
   <211> 20
   <212> DNA
   <213> Artificial Sequence
<220>
   <223> LRRK2_F6_rev
<400> 16
   aggctgctcg gtaaactgat 20
<210> 17
   <211> 20
   <212> DNA
   <213> Artificial Sequence
<220>
   <223> LRRK2_F7_For
<400> 17
   ttcctgggtt gctggagatt 20
<210> 18
   <211> 20
   <212> DNA
   <213> Artificial Sequence
<220>
   <223> LKRK2_F7_rev
<400> 18
   tcccagacac aatccagctt 20
<210> 19
   <211> 20
   <212> DNA
   <213> Artificial Sequence
<220>
   <223> LRRK2_F8_For
<400> 19
   acttctgccc aggtctttga 20
<210> 20
   <211> 22
   <212> DNA
   <213> Artificial Sequence
<220>
   <223> LRRK2_F8_rev
<400> 20
   tgtgagtacc ctttccatgt ga 22
<210> 21
   <211> 19
   <212> DNA
   <213> Artificial Sequence
<220>
   <223> LRRK2_F1_500F
<400> 21
   gatctcctcc taacttcag 19
<210> 22
   <211> 18
   <212> DNA
   <213> Artificial Sequence
<220>
   <223> LRRK2-F1-700r
<400> 22
   caatgcagca catgaagc 18
<210> 23
   <211> 20
   <212> DNA
   <213> Artificial Sequence
<220>
   <223> LRRK2_F2_500F
<400> 23
   gaagtggctg aaagtggctg 20
<210> 24
   <211> 20
   <212> DNA
   <213> Artificial Sequence
<220>
   <223> LRRK2_F2_700r
<400> 24
   gaatatcatt cttgaaacac 20
<210> 25
   <211> 19
   <212> DNA
   <213> Artificial Sequence
<220>
   <223> LRRK2_F3_500F
<400> 25
   catcagcttg ctcttaagg 19
<210> 26
   <211> 18
   <212> DNA
   <213> Artificial Sequence
<220>
   <223> LKRK2_F3_700r
<400> 26
   gaggctgttc cttcttcc 18
<210> 27
   <211> 18
   <212> DNA
   <213> Artificial Sequence
<220>
   <223> LRRK2_F5_500F
<400> 27
   cttataaccg aatgaaac 18
<210> 28
   <211> 19
   <212> DNA
   <213> Artificial Sequence
<220>
   <223> LKRK2_F5_700r
<400> 28
   ctatagaatt cctcacgac 19
<210> 29
   <211> 19
   <212> DNA
   <213> Artificial Sequence
<220>
   <223> LRRK2_F7_500F
<400> 29
   ggatcgcctg cttcagcag 19
<210> 30
   <211> 18
   <212> DNA
   <213> Artificial Sequence
<220>
   <223> LKRK2_F7_700r
<400> 30
   cattctacag cagtactg 18
<210> 31
   <211> 19
   <212> DNA
   <213> Artificial Sequence
<220>
   <223> LRRK2_F8_500F
<400> 31
   gctgctcctt tgaagatac 19
<210> 32
   <211> 19
   <212> DNA
   <213> Artificial Sequence
<220>
   <223> LRRK2_F8_700r
<400> 32
   gtctaccacc actgttatg 19
<210> 33
   <211> 57
   <212> DNA
   <213> Artificial Sequence
<220>
   <223> LRRK2 shRNA-1
<400> 33
   ccggccacaa attcaacgga aagaactcga gttctttccg ttgaatttgt ggttttt 57
<210> 34
   <211> 57
   <212> DNA
   <213> Artificial Sequence
<220>
   <223> LRRK2 shRNA-2
<400> 34
   ccggcccaaa ttggtggaac tcttactcga gtaagagttc caccaatttg ggttttt 57
<210> 35
   <211> 57
   <212> DNA
   <213> Artificial Sequence
<220>
   <223> luciferase shRNA
<400> 35
   ccggcgctga gtacttcgaa atgtcctcga ggacatttcg aagtactcag cgttttt 57